(19) Europäisches Patentamt
European Patent Office
Office européen des brevets

(11) Numéro de publication: **0 147 317**
**A2**

(12) # DEMANDE DE BREVET EUROPEEN

(21) Numéro de dépôt: 84402675.7

(22) Date de dépôt: 20.12.84

(51) Int. Cl.⁴: **C 07 D 513/04**
C 07 D 487/04, C 07 D 471/04
C 07 D 498/04, A 61 K 31/425
A 61 K 31/40, A 61 K 31/435
A 61 K 31/54, A 61 K 31/42
A 61 K 31/535
//(C07D513/04, 277:00, 209:00),
(C07D487/04, 209:00, 209:00),
(C07D471/04, 221:00, 209:00),
(C07D513/04, 279:00, 209:00)

(30) Priorité: 21.12.83 FR 8320474

(43) Date de publication de la demande:
03.07.85 Bulletin 85/27

(84) Etats contractants désignés:
AT BE CH DE FR GB IT LI LU NL SE

(71) Demandeur: RHONE-POULENC SANTE
Les Miroirs 18 Avenue d'Alsace
F-92400 Courbevoie Cedex(FR)

(72) Inventeur: Fabre, Jean-Louis
9, rue Fagon
F-75013 Paris(FR)

(72) Inventeur: Farge, Daniel
30, rue des Pins Sylvestres
F-94320 Thiais(FR)

(72) Inventeur: James, Claude
31 bis, avenue Gambetta
F-75020 Paris(FR)

(72) Inventeur: Lavé, Daniel
72 Bld de la Villette
F-75019 Paris(FR)

(74) Mandataire: Le Goff, Yves et al,
RHONE-POULENC RECHERCHES Brevets Pharma 25,
Quai Paul Doumer
F-92408 Courbevoie(FR)

(54) **Nouveaux dérivés ortho-condensés du pyrrole, leur préparation et les médicaments qui les contiennent.**

(57) Nouveaux dérivés du pyrrole de formule générale (I) dans laquelle R'= H, alcoyle ou phényle éventuellement substitué par halogène, alcoyle, alcoyloxy ou alcoylthio, Z = 0 ou S, p = 0 ou 1 et

- soit A est un hétérocycle tel qu'avec le pyrrole, il forme un cycle 1H,3H-pyrrolo [1,2-c] thiazole, dihydro-2,3 1H-pyrrolizine éventuellement substitué par OH, tétrahydro-5,6,7,8 indolizine, dihydro-1,2 4H-pyrrolo [1,2-c] thiazine-1,3, dihydro-2,3 pyrrolo [2,1-b] thiazole, 1H,3H-pyrrolo [1,2-c] oxazole, dihydro-1,2 4H-pyrrolo [1,2-c] oxazine-1,3 ou dihydro-2,3 pyrrolo [2,1-b] oxazole, n = 0 ou 1, Het = pyridyle-3 ou thiazolyle-5 et

1) ou bien R = H, halogène, alcoyle ou phényle éventuellement substitué par halogène, alcoyloxy ou alcoylthio et Y = alcoyle ou phényle éventuellement substitué par halogène, alcoyle, alcoyloxy ou alcoylthio, ou encore Y = $-NR_1R_2$ avec :

- soit $R_1$ = H et $R_2$ = alcoyle non substitué, cycloalcoyle (3 à 6 C), alcényle (2 à 4 C), alcynyle (3 à 4 C), benzyle ou phényle éventuellement substitués par halogène, alcoyle, alcoyloxy, alcoylthio, $CF_3$ ou $NO_2$, ou bien $R_2$ = adamantyle, pyridyle ou pyridylméthyle,

- soit $R_1$ et $R_2$ = tous deux alcoyle non substitué

- soit $R_1$ et $R_2$ forment un radical phényl-4-pipérazinyle-1 dont la partie phényle peut être substituée par halogène, alcoyle, alcoyloxy, alcoylthio, $CF_3$ ou $NO_2$

2) ou bien R = halogène, alcoyle ou phényle éventuellement substitué par halogène, alcoyloxy ou alcoylthio, et Y = $NH_2$

- soit A est un hétérocycle tel qu'avec le pyrrole il forme un cycle dihydro-2,3 1H-pyrrolizine substitué par OH, dihydro-2,3 pyrrolo [2,1-b] thiazole ou dihydro-2,3 pyrrolo [2,1-b] oxazole, R = H, Y = $NH_2$, n = 0 ou 1 et Het = thiazolyle-5 ou

./...



Croydon Printing Company Ltd.

pyridyle-3.

- soit A est un hétérocycle tel qu'avec le pyrrole il forme un cycle 1H,3H-pyrrolo [1,2-c] thiazole, dihydro-2,3 1H-pyrrolizine, tétrahydro-5,6,7,8 indolizine, dihydro-1,2 4H-pyrrolo [1,2-c] thiazine-1,3, 1H,3H-pyrrolo [1,2-c] oxazole ou dihydro-1,2 4H-pyrrolo [1,2-c] oxazine-1,3, R = H, Y = $NH_2$ et

- ou bien Het = thiazolyle-5 et n = 0 ou 1

- ou bien Het = pyridyle-3 et n = 1, les alcoyle ayant 1 à 4 C en chaîne droite ou ramifiée.

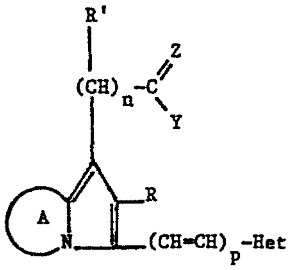

(I)

Ces produits sont utiles dans le traitement prophylactique et thérapeutique des affections thrombotiques.

La présente invention concerne de nouveaux dérivés ortho-condensés du pyrrole de formule générale :

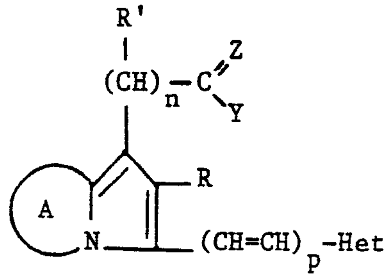

(I)

leur préparation, leurs sels et les médicaments qui les contiennent.

Dans la formule générale (I), R' représente un atome d'hydrogène ou un radical alcoyle ou phényle éventuellement substitué par un atome d'halogène ou un radical alcoyle, alcoyloxy ou alcoylthio, Z représente un atome d'oxygène ou de soufre, p représente le nombre entier zéro ou 1 et

A - soit le symbole A représente un hétérocycle tel qu'avec le noyau pyrrole auquel il est condensé, il forme un cycle 1H,3H-pyrrolo [1,2-c] thiazole, dihydro-2,3 1H-pyrrolizine éventuellement substitué par un radical hydroxy, tétrahydro-5,6,7,8 indolizine, dihydro-1,2 4H-pyrrolo [1,2-c] thiazine-1,3, dihydro-2,3 pyrrolo [2,1-b] thiazole, 1H,3H-pyrrolo [1,2-c] oxazole, dihydro-1,2 4H-pyrrolo [1,2-c] oxazine-1,3 ou dihydro-2,3 pyrrolo [2,1-b] oxazole, n représente le nombre entier 0 ou 1, Het représente un radical pyridyle-3 ou thiazolyle-5 et

1) ou bien R représente un atome d'hydrogène ou d'halogène ou un radical alcoyle ou phényle éventuellement substitué par un atome d'halogène ou un radical alcoyle, alcoyloxy ou alcoylthio et Y représente un radical alcoyle (de préférence méthyle) ou phényle éventuellement substitué par un atome d'halogène ou un radical alcoyle, alcoyloxy ou alcoylthio, ou encore Y représente un radical de formule générale :

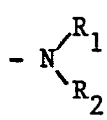

(II)

dans laquelle

- soit $R_1$ représente un atome d'hydrogène et $R_2$ représente un radical alcoyle non substitué, cycloalcoyle contenant 3 à 6 atomes de carbone, alcényle contenant 2 à 4 atomes de carbone, alcynyle contenant 3 ou 4 atomes de carbone, benzyle ou phényle éventuellement substitués par un atome d'halogène ou un radical alcoyle, alcoyloxy, alcoylthio, trifluorométhyle ou nitro, ou bien $R_2$ représente un radical adamantyle, pyridyle ou pyridylméthyle,

- soit $R_1$ et $R_2$ représentent tous deux un radical alcoyle non substitué,

- soit $R_1$ et $R_2$ forment avec l'atome d'azote auquel ils sont liés un radical phényl-4 pipérazinyle-1 dont la partie phényle peut être éventuellement substituée par un atome d'halogène ou un radical alcoyle, alcoyloxy, alcoylthio, trifluorométhyle ou nitro,

2) ou bien R représente un atome d'halogène ou un radical alcoyle ou phényle éventuellement substitué par un atome d'halogène ou un radical alcoyle, alcoyloxy ou alcoylthio, et Y représente un radical amino,

B - soit le symbole A représente un hétérocycle tel qu'avec le noyau pyrrole auquel il est condensé, il forme un cycle dihydro-2,3 1H-pyrrolizine substitué par un radical hydroxy, dihydro-2,3 pyrrolo [2,1-b] thiazole ou dihydro-2,3 pyrrolo [2,1-b] oxazole, R représente un atome d'hydrogène, Y représente un radical amino, n représente le nombre zéro ou 1 et Het représente un radical thiazolyle-5 ou pyridyle-3.

C - soit le symbole A représente un hétérocycle tel qu'avec le noyau pyrrole auquel il est condensé, il forme un cycle 1H,3H-pyrrolo [1,2-c] thiazole, dihydro-2,3 1H-pyrrolizine, tétrahydro-5,6,7,8 indolizine, dihydro-1,2 4H-pyrrolo [1,2-c] thiazine-1,3, 1H,3H-pyrrolo [1,2-c] oxazole ou dihydro-1,2 4H-pyrrolo [1,2-c] oxazine-1,3, R représente un atome d'hydrogène, Y représente un radical amino et

- soit Het représente le radical thiazolyle-5 et n est égal à zéro ou 1,

- soit Het représente le radical pyridyle-3 et n est égal à 1,

étant entendu que, sauf mention spéciale, les radicaux alcoyle et portions alcoyle contiennent 1 à 4 atomes de carbone et sont en chaîne droite ou ramifiée.

Selon l'invention, les produits de formule générale (I) dans laquelle Z représente un atome d'oxygène, Y représente un radical alcoyle ou phényle éventuellement substitué par un atome d'halogène ou un radical alcoyle, alcoyloxy ou alcoylthio et les autres symboles sont définis comme précédemment, peuvent être obtenus par action d'un dérivé organomagnésien de formule générale :

$$Y'\text{-}MgX_1 \qquad \text{(III)}$$

dans laquelle Y' représente un radical alcoyle ou phényle éventuellement substitué par un atome d'halogène ou un radical alcoyle, alcoyloxy ou alcoylthio et $X_1$ représente un atome d'halogène sur un nitrile de formule générale :

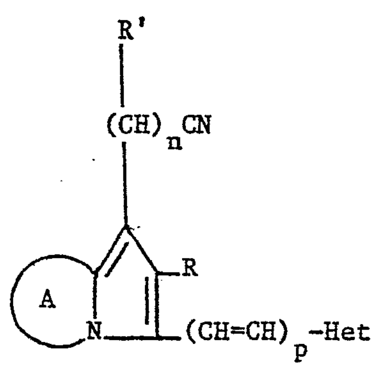

(IV)

dans laquelle les symboles sont définis comme précédemment, en opérant selon les méthodes connues de l'homme du métier pour faire réagir un dérivé organomagnésien sur un nitrile.

Les nitriles de formule générale (IV) dans laquelle n est égal à zéro et les autres symboles sont définis comme précédemment peuvent être obtenus par action d'un nitrile de formule générale :

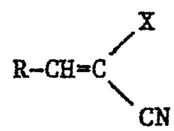

(V)

dans laquelle X représente un atome d'halogène tel que le chlore ou le brome et R est défini comme précédemment, sur un produit de formule générale :

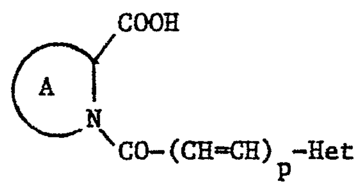

(VI)

dans laquelle les différents symboles sont définis comme précédemment.

La réaction s'effectue généralement dans l'anhydride acétique par chauffage à une température comprise entre 80 et 130°C.

Les produits de formule générale (V) peuvent être obtenus par application ou adaptation des méthodes décrites par L. LECLERCQ et A.BRUYLANTS, Bull. Soc. chim. belges, 58, 5 (1949) ou J.C. POMMELET et coll., Angew. Chem., 93, 594 (1981), ou R. CARRIE et coll., Bull. Soc. chim. France, 1963 (1976) ou H. BRINTZINGER et coll., Angew. Chem., 60, 312 (1948).

Les produits de formule générale (VI) peuvent être obtenus par condensation d'un produit de formule générale :

$$Het-(CH{=}CH)_p-COX_2 \qquad (VII)$$

dans laquelle p est défini comme précédemment et $X_2$ représente un atome d'halogène ou forme avec le radical auquel il est attaché un anhydride mixte, sur un produit de formule générale :

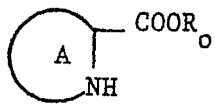

(VIII)

dans laquelle A est défini comme précédemment et $R_o$ représente un atome d'hydrogène ou un radical alcoyle, suivie d'une hydrolyse lorsque $R_o$ représente un radical alcoyle et/ou que le symbole A dans la formule générale (VI) doit représenter une pyrrolidine substituée par un radical hydroxy.

La condensation du produit de formule générale (VII) sur le produit de formule générale (VIII) s'effectue généralement dans un solvant organique inerte tel que le chloroforme en présence d'un accepteur d'acide tel que la triéthylamine à une température comprise entre 0 et 65°C.

Lorsque $R_o$ représente un radical alcoyle et/ou que A représente une pyrrolidine substituée par un radical hydroxy, l'hydrolyse s'effectue par toute méthode connue de l'homme du métier pour transformer un ester en acide et/ou en alcool sans toucher au reste de la molécule, notamment par chauffage en milieu alcalin dans l'eau ou dans un solvant hydro-alcoolique tel que le mélange eau-éthanol à une température comprise entre 20 et 80°C.

Les produits de formule générale (VIII) peuvent être obtenus par application ou adaptation des méthodes décrites par J.C.WRISTON et C.G. Mc KENZIE, J. Biol. Chem., 225, 607 (1957) ou S. WOLFF, G. MILITELLO et coll., Tet. Letters, 3913 (1979) ou R.L.JOHNSON, E.E. SMISSMAN et N.P. PLOTNIKOFF, J. Med. Chem., 21, 165 (1978).

Lorsque A représente un hétérocycle dans lequel est présent un atome d'oxygène, on n'isole pas le produit de formule générale (VIII) mais on obtient directement le produit de formule générale (VI), la condensation du produit de formule générale (VII) s'effectuant in situ dans le mélange réactionnel.

Les nitriles de formule générale (IV) dans laquelle n est égal à 1 et les autres symboles sont définis comme précédemment peuvent être obtenus par condensation de l'isocyanure de tosylméthyle de formule :

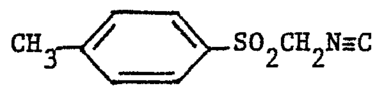

(IX)

sur un dérivé carbonylé de formule générale :

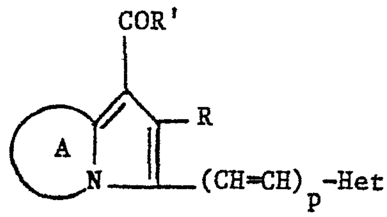

(X)

dans laquelle les symboles sont définis comme précédemment.

La condensation s'effectue généralement en deux étapes successives : a) en opérant dans un solvant tel que le diméthoxy-1,2 éthane, le diméthylsulfoxyde ou l'hexaméthylphosphoramide en présence d'une base telle que le tert-butylate de potassium à une température comprise entre -60°C et 0°C, puis b) en poursuivant la réaction à une température comprise entre 0°C et la température de reflux du mélange réactionnel après avoir ajouté un alcool tel que le méthanol.

Les dérivés carbonylés de formule générale (X) peuvent être préparés par action d'un produit de formule générale :

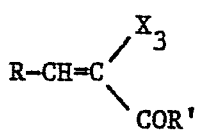

(XI)

dans laquelle R et R' sont définis comme précédemment et $X_3$ représente un atome d'halogène tel que le chlore ou le brome, sur un produit de formule générale (VI) tel que défini précédemment.

On opère généralement dans l'anhydride acétique à une température comprise entre 80 et 130°C.

Les produits de formule générale (XI) peuvent être préparés par application ou adaptation des méthodes décrites par H.SCHULZ et H.WAGNER, Angew. Chem., 62,. 105 (1950) ou H. HIBBERT, E.O.HOUGHTON et K.A. TAYLOR, J. Amer. Chem. Soc., 51, 611 (1929).

Les dérivés carbonylés de formule générale (X) dans laquelle A est défini comme précédemment, p est égal à 0 et R' représente un atome d'hydrogène peuvent également être préparés par formylation d'un produit de formule générale :

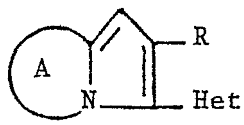 (XII)

dans laquelle A, R et Het sont définis comme précédemment.

On effectue généralement la formylation par tout moyen connu de l'homme du métier pour formyler un noyau pyrrole sans toucher au reste de la molécule, notamment au moyen d'un mélange de chlorure de phosphoryle et de diméthylformamide, à une température comprise entre 0 et 20°C.

Les produits de formule générale (XII) peuvent être préparés par décarboxylation d'un acide de formule générale :

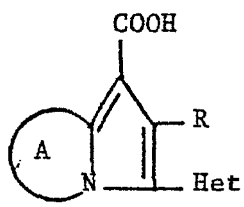 (XIII)

dans laquelle A, Het et R sont définis comme précédemment, selon les méthodes connues en soi pour décarboxyler un acide, par exemple par chauffage en présence de poudre de cuivre.

Les acides de formule générale (XIII) peuvent être préparés par hydrolyse des nitriles de formule générale (IV) dans laquelle p et n représentent le nombre zéro et les autres symboles sont définis comme précédemment.

L'hydrolyse des nitriles de formule générale (IV) peut être effectuée par toute méthode connue de l'homme du métier pour transformer un nitrile en acide sans toucher aur reste de la molécule. Il est généralement avantageux d'effectuer l'hydrolyse en milieu basique dans un alcool à haut point d'ébullition, par exemple au moyen de potasse dans l'éthylèneglycol entre 100°C et le reflux du mélange réactionnel.

Selon l'invention, les produits de formule générale (I) dans laquelle les symboles sont définis comme précédemment, à l'exception pour Z de représenter un atome de soufre et pour Y de représenter un radical alcoyle ou phényle éventuellement substitué par un atome d'halogène ou un radical alcoyle, alcoyloxy ou alcoylthio, peuvent être préparés par action de l'ammoniac ou d'une amine de formule générale :

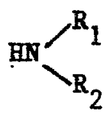

(XIV)

dans laquelle $R_1$ et $R_2$ sont définis comme précédemment sur un acide de formule générale :

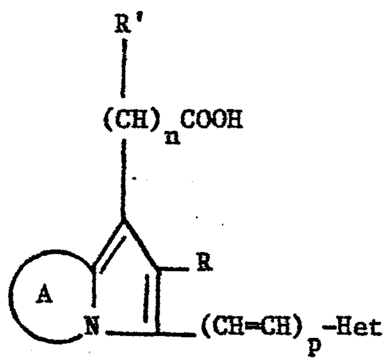

(XV)

dans laquelle les différents symboles sont définis comme précédemment.

Il est particulièrement avantageux d'utiliser l'acide de formule générale (XV) sous une forme activée telle que le chlorure d'acide ou de le faire réagir avec le N,N'-carbonyldiimidazole ou un chloroformiate d'alcoyle avant de faire réagir l'ammoniac ou l'amine de formule générale (XIV).

Généralement il est préférable de faire réagir le chlorure d'acide et d'effectuer la réaction dans un solvant organique tel que le chloroforme ou le chlorure de méthylène à une température comprise entre 0°C et le reflux du mélange réactionnel.

Les acides de formule générale (XV) peuvent être préparés par hydrolyse des nitriles de formule générale (IV), dans laquelle les différents symboles sont définis comme précédemment. L'hydrolyse des nitriles de formule générale (IV) peut être effectuée par toute méthode connue de l'homme du métier pour transformer un nitrile en acide sans toucher au reste de la molécule. Il est généralement avantageux d'effectuer l'hydrolyse en milieu basique dans un alcool à haut point d'ébullition, par exemple au moyen de potasse dans l'éthylèneglycol entre 100°C et le reflux du mélange réactionnel.

Les acides de formule générale (XV) dans laquelle R représente un atome d'halogène, p est égal à 0 et les autres symboles sont définis comme précédemment peuvent également être obtenus par halogénation d'un produit de formule générale (XV) dans laquelle R représente un atome d'hydrogène, p est égal à 0 et les autres symboles sont définis comme précédemment en opérant par toute méthode connue de l'homme du métier permettant d'effectuer la réaction désirée sans toucher au reste de la molécule.

Selon l'invention, les produits de formule générale (I) dans laquelle Z représente un atome d'oxygène, Y représente un radical méthyle et les autres symboles sont définis comme précédemment peuvent être obtenus par action de l'éthoxymagnésien du malonate d'éthyle sur un halogénure d'acide de formule générale :

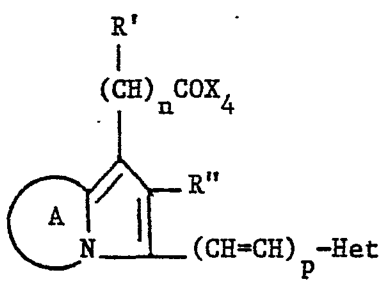

(XVI)

dans laquelle $X_4$ représente un atome d'halogène, R" a la définition donnée précédemment pour R et les autres symboles sont définis comme précédemment, en opérant selon les méthodes connues de l'homme du métier pour faire réagir l'éthoxymagnésien du malonate d'éthyle sur un halogénure d'acide.

Les produits de formule générale (XVI) peuvent être obtenus à partir des acides de formule générale (XV) définie comme précédemment par toute méthode connue de l'homme du métier pour

transformer un acide en halogénure d'acide sans toucher au reste de la molécule.

Selon l'invention les produits de formule générale (I) dans laquelle Z représente un atome de soufre et les autres symboles sont définis comme précédemment peuvent être préparés par thionation d'un produit de formule générale (I) dans laquelle Z représente un atome d'oxygène et les autres symboles sont définis comme précédemment, c'est-à-dire un produit de formule générale :

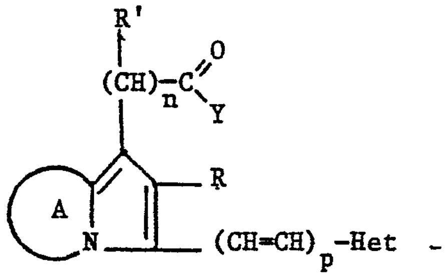

(XVII)

La réaction s'effectue généralement au moyen d'un réactif de thionation tel que le pentasulfure de phosphore dans un solvant organique tel que le toluène, le dioxanne ou la pyridine, à une température voisine de 100°C ou au moyen du réactif de LAWESSON [bis (méthoxy-4 phényl)-2,4 dithioxo-2,4 dithia-1,3 diphospha-2,4 étanne] dans un solvant organique tel que le toluène à une température voisine de 50°C ou le diméthoxy-1,2 éthane ou l'hexaméthylphosphoramide à une température voisine de 20°C.

Selon l'invention, les produits de formule générale (I) dans laquelle Z représente un atome de soufre, Y représente un radical amino, p est égal à 0 et les autres symboles sont définis comme précédemment peuvent être préparés à partir des nitriles de formule générale (IV) dans laquelle les symboles ont la définition correspondante, par toute méthode connue de l'homme du métier pour passer d'un nitrile à un thioamide sans toucher au reste de la molécule. Il est particulièrement avantageux de faire agir sur le nitrile de formule générale (IV) le sulfure d'hydrogène dans un solvant tel que la pyridine en présence de triéthylamine, en opérant à une température comprise entre 0 et 50°C.

Selon l'invention, les produits de formule générale (I) dans laquelle les symboles sont définis comme précédemment en A2), en B ou en C peuvent être préparés par hydrolyse d'un nitrile de

formule générale (IV) dans laquelle les symboles ont les définitions correspondantes.

L'hydrolyse peut être effectuée par tout moyen connu de l'homme du métier pour transformer un nitrile en amide sans toucher au reste de la molécule, notamment par chauffage en milieu alcalin dans un solvant organique tel que le tert-butanol à une température comprise entre 30 et 85°C, ou en milieu acide concentré à une température comprise entre 20 et 100°C.

Selon l'invention, les produits de formule générale (I) dans laquelle Z représente un atome d'oxygène, n est égal à zéro et les autres symboles sont définis comme précédemment peuvent être préparés par action d'un produit de formule générale :

R-C≡C-CO-Y (XVIII)

dans laquelle les symboles sont définis comme précédemment, sur un produit de formule générale (VI) défini comme précédemment.

On opère généralement dans l'anhydride acétique par chauffage à une température comprise entre 80 et 130°C.

Les produits de formule générale (XVIII) peuvent être préparés par application ou adaptation de la méthode décrite par W.D.CROW et N.J. LEONARD, J. Org. Chem., 30, 2660 (1965).

Selon l'invention, les produits de formule générale (I) dans laquelle R représente un atome d'halogène, p est égal à 0 et les autres symboles sont définis comme précédemment peuvent également être préparés par halogénation d'un produit de formule générale (I) dans laquelle R représente un atome d'hydrogène, p est égal à 0 et les autres symboles sont définis comme précédemment, en opérant par toute méthode connue de l'homme du métier permettant d'effectuer la réaction sans toucher au reste de la molécule, par exemple par action d'un halogène dans un solvant tel que l'acide acétique.

Lorsque, dans les différents procédés décrits ci-dessus, certaines réactions sont incompatibles avec des fonctions présentes dans la molécule, il est bien entendu que ces fonctions doivent être protégées au préalable par blocage. Le blocage et le déblocage subséquent peuvent se faire par toute méthode connue de l'homme du métier.

Les nouveaux produits de formule générale (I) peuvent être purifiés par les méthodes connues habituelles, par exemple par cristallisation, chromatographie ou extractions successives en milieu acide et basique.

Les nouveaux produits de formule générale (I) peuvent être transformés en sel d'addition avec les acides par action d'un acide dans un solvant organique tel qu'un alcool, une cétone, un éther ou un solvant chloré. Le sel formé précipite, éventuellement après concentration de sa solution; il est séparé par filtration ou décantation.

Les nouveaux produits de formule générale (I) et leurs sels présentent d'intéressantes propriétés pharmacologiques les rendant utiles dans le traitement prophylactique et thérapeutique des affections thrombotiques. Ils se sont montrés actifs chez la souris à des doses inférieures à 100 mg/kg par voie orale dans le test de génération de thromboxane $A_2$ sérique selon la technique de R.J. FLOWER et coll. [Brit. J. Pharmacol. 74 (4) 791 P (1981)].

Les nouveaux produits de formule générale (I) et leurs sels présentent en outre une toxicité faible. Leur $DL_{50}$ est généralement comprise entre 300 et 900 mg/kg, par voie orale chez la souris.

Pour l'emploi médicinal, il peut être fait usage des nouveaux produits de formule générale (I) tels quels ou à l'état de sels pharmaceutiquement acceptables, c'est-à-dire non toxiques aux doses d'utilisation.

Comme exemples de sels pharmaceutiquement acceptables on peut citer les sels d'addition avec les acides minéraux tels que chlorhydrates, sulfates, nitrates, phosphates ou organiques tels que acétates, propionates, succinates, benzoates, fumarates, maléates, méthanesulfonates, iséthionates, théophylline-acétates, salicylates, phénolphtalinates, méthylène-bis-β-oxynaphtoates ou des dérivés de substitution de ces composés.

Sont particulièrement intéressants les produits de formule générale (I) dans laquelle R' représente un atome d'hydrogène, Z représente un atome d'oxygène, p est égal à zéro et

A - soit le symbole A représente un hétérocycle tel qu'avec le noyau pyrrole auquel il est condensé, il forme un cycle 1H,3H-pyrrolo [1,2-c] thiazole, dihydro-2,3 1H-pyrrolizine, tétrahydro-5,6,7,8 indolizine, dihydro-1,2 4H-pyrrolo [1,2-c] thiazine-1,3, n est égal à zéro, Het représente un radical pyridyle-3 et

1) ou bien R représente un atome d'hydrogène et Y représente un radical alcoyle (de préférence méthyle) ou phényle, ou encore Y représente un radical de formule générale (II) dans laquelle :

- soit $R_1$ représente un atome d'hydrogène et $R_2$ représente un radical alcoyle non substitué, cycloalcoyle contenant 3 à 6 atomes de carbone, alcényle contenant 2 à 4 atomes de carbone, benzyle ou phényle éventuellement substitués par un atome d'halogène ou un radical alcoyloxy, trifluorométhyle ou bien $R_2$ représente un radical adamantyle, pyridyle ou pyridylméthyle,

- soit $R_1$ et $R_2$ représentent tous deux un radical alcoyle non substitué

- soit $R_1$ et $R_2$ forment avec l'atome d'azote auquel ils sont liés un radical phényl-4 pipérazinyle-1

2) ou bien R représente un atome d'halogène ou un radical alcoyle, et Y représente un radical amino,

B - soit le symbole A représente un hétérocylcle tel qu'avec le noyau pyrrole auquel il est condensé il forme un cycle dihydro-2,3 1H-pyrrolizine substitué par un radical hydroxy, dihydro-2,3 pyrrolo [2,1-b] thiazole, R représente un atome d'hydrogène, Y représente un radical amino, n est égal à zéro et Het représente un radical pyridyle-3.

C - soit le symbole A représente un hétérocycle tel qu'avec le noyau pyrrole auquel il est condensé, il forme un cycle 1H,3H-pyrrolo [1,2-c] thiazole, R représente un atome d'hydrogène, Y représente un radical amino et

- soit Het représente un radical thiazolyle-5 et n est égal à zéro
- soit Het représente un radical pyridyle-3 et n est égal à 1.

Sont plus particulièrement intéressants les produits de formule générale (I) dans laquelle R' représente un atome d'hydrogène, Z représente un atome d'oxygène, p est égal à zéro et

A - soit le symbole A représente un hétérocycle tel qu'avec le noyau pyrrole auquel il est condensé, il forme un cycle 1H,3H-pyrrolo [1,2-c] thiazole, dihydro-1,2 4H-pyrrolo [1,2-c] thiazine-1,3, n est égal à zéro, Het représente un radical pyridyle-3 et

1) ou bien R représente un atome d'hydrogène et Y représente un radical phényle, ou encore Y représente un radical de formule générale (II) dans laquelle $R_1$ représente un atome d'hydrogène et $R_2$ représente un radical alcoyle non substitué, benzyle ou phényle éventuellement substitué par un atome d'halogène ou un radical alcoyloxy, ou bien $R_2$ représente un radical adamantyle ou pyridyle,

2) ou bien R représente un atome d'halogène ou un radical alcoyle, et Y représente un radical amino,

B - soit le symbole A représente un hétérocycle tel qu'avec le noyau pyrrole auquel il est condensé il forme un cycle dihydro-2,3 pyrrolo [2,1-b] thiazole, R représente un atome d'hydrogène, Y représente un radical amino, n est égal à zéro et Het représente un radical pyridyle-3.

Sont d'un intérêt tout particulier, le produits suivants :

- N-(Adamantyl-1) (pyridyl-3)-5 1H,3H pyrrolo [1,2-c] thiazolecarboxamide-7
- N-(chloro-3 phényl) (pyridyl-3)-5 1H,3H-pyrrolo [1,2-c] thiazolecarboxamide-7
- Benzoyl-7 (pyridyl-3)-5 1H,3H-pyrrolo [1,2-c] thiazole
- N-Isopropyl (pyridyl-3)-5 1H,3H-pyrrolo [1,2-c] thiazolecarboxamide-7
- N-Benzyl (pyridyl-3)-5 1H,3H-pyrrolo [1,2-c] thiazolecarboxamide-7
- Bromo-6 (pyridyl-3)-5 1H,3H-pyrrolo [1,2-c] thiazolecarboxamide-7
- N-(Pyridyl-3)(pyridyl-3)-5 1H,3H-pyrrolo [1,2-c] thiazolecarboxamide-7

- N-Méthyl (pyridyl-3)-5 dihydro-1,2 4H-pyrrolo [1,2-c] thiazine-1,3 carboxamide-8
- Méthyl-7 (pyridyl-3)-6 dihydro-1,2 4H-pyrrolo [1,2-c] thiazine-1,3 carboxamide-8
- N-Phényl (pyridyl-3)-6 dihydro-1,2 4H-pyrrolo [1,2-c] thiazine-1,3 carboxamide-8
- N-(Méthoxy-3 phényl) (pyridyl-3)-5 1H,3H-pyrrolo [1,2-c] thiazolecarboxamide-7
- (Pyridyl-3)-5 dihydro-2,3 pyrrolo [2,1-b] thiazolecarboxamide-7
- N-Méthyl (pyridyl-3)-5 1H,3H-pyrrolo [1,2-c] thiazolecarboxamide-7.

Les exemples suivants, donnés à titre non limitatif, montrent comment l'invention peut être mise en pratique :

EXEMPLE 1 -

Une suspension de 14,6 g de chlorhydrate de chloroformyl-7 (pyridyl-3)-5 1H,3H-pyrrolo[1,2-c] thiazole dans 200 cm3 de chlorure de méthylène est saturée par un courant de monométhylamine anhydre en maintenant la température du mélange réactionnel voisine de 25°C pendant 2 heures et 10 minutes. La suspension obtenue est agitée à une température voisine de 20°C pendant 16 heures. Les cristaux sont séparés par filtration, lavés 3 fois par 90 cm3 au total de chlorure de méthylène et mis en suspension dans 100 cm3 d'une solution aqueuse de soude 2 N. Les cristaux sont séparés par filtration, lavés 5 fois par 250 cm3 au total d'eau distillée et 3 fois par 150 cm3 au total d'acétone et séchés sous pression réduite (20 mm de mercure ; 2,7 kPa) à une température voisine de 20°C en présence de potasse en pastilles. On obtient ainsi 11,3 g de produit fondant à 252°C. Ce produit est dissous dans 135 cm3 de diméthylformamide à une température voisine de 100°C. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 20 cm3 au total de diméthylformamide, 3 fois par 150 cm3 au total d'acétone et séchés sous pression réduite (20 mm de mercure ; 2,7 kPa) à une température voisine de 20°C en présence de potasse en pastilles. On obtient ainsi 9,4 g de N-méthyl (pyridyl-3)-5 1H,3H-pyrrolo[1,2-c] thiazolecarboxamide-7 sous forme de cristaux crème fondant à 254°C.

Le chlorhydrate de chloroformyl-7 (pyridyl-3)-5 1H,3H-pyrrolo[1,2-c] thiazole peut être préparé de la façon suivante :

Une suspension de 8,8 g d'acide (pyridyl-3)-5 1H,3H-pyrrolo[1,2-c] thiazolecarboxylique-7 dans un mélange de 6,25 cm3 de chlorure de thionyle, 0,05 cm3 de diméthylformamide et de 100 cm3 de dichloro-1,2 éthane est chauffée au reflux sous agitation pendant 2 heures et 30 minutes. Le mélange réactionnel est refroidi à une température voisine de 20°C et concentré à sec sous

pression réduite (20 mm de mercure ; 2,7 kPa) à une température voisine de 60°C. Le résidu obtenu est mis en suspension dans 150 cm3 de cyclohexane et le solvant est évaporé sous pression réduite (20 mm de mercure ; 2,7 kPa) à une température voisine de 60°C. La même opération est répétée 2 fois. On obtient ainsi 10 g de chlorhydrate de chloroformyl-7 (pyridyl-3)-5 pyrrolo[1,2-c] thiazole sous forme de cristaux crème fondant à 220°C.

L'acide (pyridyl-3)-5 1H,3H-pyrrolo[1,2-c] thiazolecarboxylique-7 peut être préparé de la façon suivante :

Un mélange de 18,7 g de (pyridyl-3)-5 1H,3H-pyrrolo[1,2-c] thiazolecarbonitrile-7, 16,3 g de potasse en pastilles et 160 cm3 d'éthylèneglycol est chauffé sous agitation à une température voisine de 155°C pendant 2 heures. Après 16 heures d'agitation à une température voisine de 20°C, le solvant est évaporé sous pression réduite (2 mm de mercure ; 0,27 kPa) à une température voisine de 100°C. Le résidu est dissous dans 100 cm3 d'eau distillée et la solution obtenue est amenée à un pH voisin de 5 par addition d'une solution aqueuse d'acide chlorhydrique 2 N. Les cristaux apparus sont séparés par filtration, lavés 3 fois par 150 cm3 au total d'eau distillée puis 3 fois par 150 cm3 au total d'acétone et séchés sous pression réduite (20 mm de mercure ; 2,7 kPa) à une température voisine de 20°C en présence de potasse en pastilles. On obtient ainsi 17,7 g de produit brut fondant à 264°C. Ce produit est réuni à 1,3 g de produit préparé de la même façon dans une opération antérieure et dissous dans un mélange de 650 cm3 de butanol-1 et de 150 cm3 de diméthylformamide préalablement chauffé à une température voisine de 115°C. On ajoute 0,5 g de noir décolorant à la solution obtenue et on filtre à chaud. Le filtrat est refroidi à une température voisine de 4°C pendant 16 heures. Les cristaux apparus sont séparés par filtration, lavés 2 fois par 50 cm3 au total de diméthylformamide, 3 fois par 150 cm3 au total d'éthanol, 3 fois par 150 cm3 au total d'oxyde d'isopropyle puis 3 fois par 150 cm3 au total d'éther diéthylique et séchés sous pression réduite (20 mm de mercure ; 2,7 kPa) à une température voisine de 20°C en présence de potasse en pastilles. On obtient ainsi 16,1 g de produit fondant à

266°C. Ce produit est mis en suspension dans 250 cm3 d'eau distillée et la suspension est agitée à une température voisine de 20°C pendant 2 heures. Les cristaux sont séparés par filtration, lavés 5 fois par 150 cm3 au total d'eau distillée, 3 fois par 90 cm3 au total d'éthanol, 3 fois par 90 cm3 au total d'oxyde d'isopropyle puis 3 fois par 90 cm3 au total d'éther diéthylique et séchés sous pression réduite (20 mm de mercure ; 2,7 kPa) à une température voisine de 100°C en présence de potasse en pastilles. On obtient ainsi 15,5 g d'acide (pyridyl-3)-5 1H,3H-pyrrolo[1,2-c] thiazolecarboxylique-7 sous forme de cristaux crème fondant à 266°C.

Le (pyridyl-3)-5 1H,3H-pyrrolo[1,2-c] thiazolecarbonitrile-7 peut être préparé de la façon suivante :

Une suspension de 403 g d'acide N-nicotinoylthiazolidinecarboxylique-4 dans un mélange de 1350 cm3 de chloro-2 acrylonitrile et de 1750 cm3 d'anhydride acétique est chauffée à 90°C pendant 2 heures et 40 minutes. Durant cette période, on observe un passage par une phase homogène limpide au bout de 30 minutes, suivi d'une précipitation 10 minutes plus tard. Après refroidissement à une température voisine de 4°C pendant 16 heures, les cristaux apparus sont séparés par filtration, lavés 2 fois par 200 cm3 au total d'anhydride acétique, 3 fois par 300 cm3 au total d'acétone et séchés sous pression réduite (20 mm de mercure ; 2,7 kPa) à une température voisine de 20°C en présence de potasse en pastilles. Le produit ainsi obtenu est mis en suspension dans 2400 cm3 d'une solution aqueuse de soude 2 N. Après agitation à une température voisine de 20°C pendant 1 heure et 30 minutes, les cristaux apparus sont séparés par filtration, lavés 5 fois par 1250 cm3 au total d'eau distillée, 3 fois par 1200 cm3 au total d'éthanol, 3 fois par 900 cm3 au total d'éther diéthylique et séchés sous pression réduite (20 mm de mercure ; 2,7 kPa) à une température voisine de 20°C en présence de potasse en pastilles. On obtient ainsi 159,7 g de (pyridyl-3)-5 1H,3H-pyrrolo[1,2-c] thiazolecarbonitrile-7 sous forme de cristaux crème fondant à 170°C.

L'acide N-nicotinoylthiazolidinecarboxylique-4 peut être obtenu de la façon suivante :

A une solution de 400 g d'acide thiazolidinecarboxylique-4 et de 613 g de triéthylamine dans 4500 cm3 de chloroforme, on ajoute en 1 heure à une température comprise entre 30 et 52°C, 534 g de chlorhydrate de chlorure de nicotinoyle. La solution obtenue est chauffée à une température voisine de 64°C pendant 4 heures. Après agitation à une température voisine de 20°C pendant 16 heures, les cristaux apparus sont séparés par filtration, lavés 3 fois par 1500 cm3 au total de chloroforme puis 3 fois par 1500 cm3 au total d'éther diéthylique et séchés sous pression réduite (20 mm de mercure ; 2,7 kPa) à une température voisine de 20°C en présence de potasse en pastilles. On obtient ainsi 403 g d'acide N-nicotinoylthiazolidinecarboxylique-4 sous forme de cristaux blancs fondant à 190°C.

EXEMPLE 2 -

Une suspension de 17,5 g de chlorhydrate de chloroformyl-7 (pyridyl-3)-5 1H,3H-pyrrolo[1,2-c] thiazole dans 360 cm3 de chlorure de méthylène est saturée en 3 heures et 30 minutes par un courant d'éthylamine anhydre à une température comprise entre 17°C et 30°C. La solution obtenue est agitée à une température voisine de 20°C pendant 16 heures puis est additionnée de 300 cm3 de chlorure de méthylène et de 300 cm3 d'eau distillée. La phase organique est séparée par décantation, lavée 2 fois par 600 cm3 au total d'eau distillée, séchée sur du sulfate de magnésium anhydre, additionnée de 0,5 g de noir décolorant, filtrée et concentrée à sec sous pression réduite (20 mm de mercure ; 2,7 kPa) à une température voisine de 50°C. On obtient ainsi 12,5 g de produit fondant à 165°C. Ce produit est dissous dans 100 cm3 d'éthanol bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 10 cm3 au total d'éthanol refroidi à une température voisine de 4°C et 3 fois par 75 cm3 au total d'éther

diéthylique et séchés sous pression réduite (20 mm de mercure ; 2,7 kPa) à une température voisine de 20°C en présence de potasse en pastilles. On obtient ainsi 8,3 g de produit fondant à 176°C. Ce produit est dissous dans 225 cm3 d'acétonitrile bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 3 fois par 30 cm3 au total d'acétonitrile et 3 fois par 75 cm3 au total d'éther diéthylique puis séchés sous pression réduite (20 mm de mercure ; 2,7 kPa) à une température voisine de 20°C en présence de potasse en pastilles. On obtient ainsi 7,4 g de N-éthyl (pyridyl-3)-5 1H,3H-pyrrolo[1,2-c] thiazolecarboxamide-7 sous forme de cristaux jaune clair fondant à 178°C.

Le chlorhydrate de chloroformyl-7 (pyridyl-3)-5 1H,3H-pyrrolo[1,2-c] thiazole est préparé comme à l'exemple 1.

EXEMPLE 3 -

Une suspension de 14,5 g de chlorhydrate de chloroformyl-7 (pyridyl-3)-5 1H,3H-pyrrolo[1,2-c] thiazole dans 240 cm3 de chlorure de méthylène est additionnée en 15 minutes à une température comprise entre 21°C et 34°C d'une solution de 8,5 g d'isopropylamine dans 60 cm3 de chlorure de méthylène. La solution obtenue est agitée à une température voisine de 20°C pendant 16 heures puis est additionnée de 300 cm3 de chlorure de méthylène et de 250 cm3 d'eau distillée. La phase organique est séparée par décantation, lavée 3 fois par 750 cm3 au total d'eau distillée, séchée sur du sulfate de magnésium anhydre, additionnée de 0,5 g de noir décolorant, filtrée et concentrée à sec sous pression réduite (20 mm de mercure ; 2,7 kPa) à une température voisine de 50°C. On obtient ainsi 13,5 g de produit fondant à 200°C. Ce produit est dissous dans 300 cm3 d'acétonitrile bouillant. La solution obtenue est additionnée de 0,5 g de noir décolorant et filtrée à chaud. Le filtrat est refroidi

à une température voisine de 4°C pendant 2 heures. Les cristaux apparus sont séparés par filtration, lavés 2 fois par 50 cm3 au total d'acétonitrile refroidi à une température voisine de 4°C, 2 fois par 100 cm3 au total d'éther diéthylique et séchés sous pression réduite (20 mm de mercure ; 2,7 kPa) à une température voisine de 20°C en présence de potasse en pastilles. On obtient ainsi 8,2 g de N-isopropyl (pyridyl-3)-5 1H,3H-pyrrolo[1,2-c] thiazolecarboxamide-7 sous forme de cristaux crème fondant à 218°C.

Le chlorhydrate de chloroformyl-7 (pyridyl-3)-5 1H,3H-pyrrolo[1,2-c] thiazole est préparé comme à l'exemple 1.

EXEMPLE 4 -

Une suspension de 12 g de chlorhydrate de chloroformyl-7 (pyridyl-3)-5 1H,3H-pyrrolo[1,2-c] thiazole dans 200 cm3 de chlorure de méthylène est additionnée en 15 minutes à une température comprise entre 22°C et 32°C d'une solution de 8,8 g de butylamine dans 50 cm3 de chlorure de méthylène. La solution obtenue est agitée à une température voisine de 20°C pendant 16 heures puis additionnée de 250 cm3 de chlorure de méthylène et de 200 cm3 d'eau distillée. La phase organique est séparée par décantation, lavée 2 fois par 400 cm3 au total d'eau distillée, séchée sur du sulfate de magnésium anhydre, additionnée de 0,5 g de noir décolorant, filtrée et concentrée à sec sous pression réduite (20 mm de mercure ; 2,7 kPa) à une température voisine de 50°C. On obtient ainsi 9 g de produit fondant à 140°C. Ce produit est dissous dans 80 cm3 d'acétonitrile bouillant. La solution obtenue est additionnée de 0,5 g de noir décolorant et filtrée à chaud. Le filtrat est refroidi à une température voisine de 4°C pendant 2 heures. Les cristaux apparus sont séparés par filtration, lavés 2 fois par 30 cm3 au total d'acétonitrile refroidi à une température voisine de 4°C, 3 fois par 75 cm3 au total d'éther diéthylique et séchés sous pression réduite (20 mm de mercure ; 2,7 kPa) à une température voisine de 20°C en présence de potasse en pastilles. On obtient ainsi 7 g de N-butyl

(pyridyl-3)-5 1H,3H-pyrrolo[1,2-c] thiazolecarboxamide-7 sous forme de cristaux crème fondant à 144°C.

Le chlorhydrate de chloroformyl-7 (pyridyl-3)-5 1H,3H-pyrrolo[1,2-c] thiazole est préparé comme à l'exemple 1.

EXEMPLE 5 -

Une suspension de 12 g de chlorhydrate de chloroformyl-7 (pyridyl-3)-5 1H,3H-pyrrolo[1,2-c] thiazole dans 200 cm3 de chlorure de méthylène est saturée par un courant de diméthylamine anhydre en maintenant la température du mélange réactionnel voisine de 25°C pendant 16 heures. La solution obtenue est additionnée de 250 cm3 de chlorure de méthylène et de 100 cm3 d'une solution aqueuse de soude 2 N. La phase organique est séparée par décantation, lavée 2 fois par 200 cm3 au total d'une solution aqueuse de soude 2 N et 3 fois par 600 cm3 au total d'eau distillée, séchée sur du carbonate de potassium anhydre, additionnée de 0,5 g de noir décolorant, filtrée et concentrée à sec sous pression réduite (20 mm de mercure ; 2,7 kPa) à une température voisine de 50°C. On obtient ainsi 10,7 g de produit. Ce produit est dissous dans 450 cm3 d'acétonitrile bouillant. La solution obtenue est additionnée de 0,5 g de noir décolorant et filtrée à chaud. Le filtrat est refroidi à une température voisine de 20°C pendant 1 heure. Les cristaux apparus sont séparés par filtration, lavés 3 fois par 90 cm3 au total d'acétonitrile, 3 fois par 150 cm3 au total d'oxyde d'isopropyle et séchés sous pression réduite (20 mm de mercure ; 2,7 kPa) à une température voisine de 20°C en présence de potasse en pastilles. On obtient ainsi 7,5 g de N,N-diméthyl (pyridyl-3)-5 1H,3H-pyrrolo[1,2-c] thiazolecarboxamide-7 sous forme de cristaux blancs fondant à 200°C.

Le chlorhydrate de chloroformyl-7 (pyridyl-3)-5 1H,3H-pyrrolo[1,2-c] thiazole est préparé comme à l'exemple 1.

EXEMPLE 6 -

Une suspension de 12 g de chlorhydrate de chloroformyl-7 (pyridyl-3)-5 1H,3H-pyrrolo[1,2-c] thiazole dans 200 cm3 de chlorure de méthylène est additionnée en 15 minutes à une température comprise entre 22°C et 29°C d'une solution de 12,9 g de benzylamine dans 50 cm3 de chlorure de méthylène. La suspension obtenue est agitée à une température voisine de 20°C pendant 16 heures puis additionnée de 250 cm3 de chlorure de méthylène et de 250 cm3 d'eau distillée. La phase organique est séparée par décantation, lavée 3 fois par 750 cm3 au total d'eau distillée, séchée sur du sulfate de magnésium anhydre, additionnée de 0,5 g de noir décolorant, filtrée et concentrée à sec sous pression réduite (20 mm de mercure ; 2,7 kPa) à une température voisine de 50°C. On obtient ainsi 10,8 g de produit fondant à 130°C. Ce produit est dissous dans 140 cm3 d'acétonitrile bouillant. La solution obtenue est additionnée de 0,5 g de noir décolorant et filtrée à chaud. Le filtrat est refroidi à une température voisine de 4°C pendant 2 heures. Les cristaux apparus sont séparés par filtration, lavés 2 fois par 30 cm3 au total d'acétonitrile refroidi à une température voisine de 4°C, 3 fois par 75 cm3 au total d'éther diéthylique et séchés sous pression réduite (20 mm de mercure ; 2,7 kPa) à une température voisine de 20°C en présence de potasse en pastilles. On obtient ainsi 7 g de N-benzyl (pyridyl-3)-5 1H,3H-pyrrolo[1,2-c] thiazolecarboxamide-7 sous forme de cristaux jaune pâle fondant à 150°C.

Le chlorhydrate de chloroformyl-7 (pyridyl-3)-5 1H,3H-pyrrolo[1,2-c] thiazole est préparé comme à l'exemple 1.

EXEMPLE 7 -

Une suspension de 14,5 g de chlorhydrate de chloroformyl-7 (pyridyl-3)-5 1H,3H-pyrrolo[1,2-c] thiazole dans 240 cm3 de chlorure de méthylène est additionnée en 15 minutes à une température comprise entre 21°C et 33°C d'une solution de 8,2 g de cyclopropylamine dans 60 cm3 de chlorure de méthylène. La solution

obtenue est agitée à une température voisine de 20°C pendant 16 heures ; un produit précipite. La suspension obtenue est additionnée de 300 cm3 de chlorure de méthylène et de 250 cm3 d'eau distillée. La phase organique est séparée par décantation, lavée 3 fois par 750 cm3 au total d'eau distillée, séchée sur du sulfate de magnésium anhydre, additionnée de 0,5 g de noir décolorant, filtrée et concentrée à sec sous pression réduite (20 mm de mercure ; 2,7 kPa) à une température voisine de 50°C. On obtient ainsi 13,5 g de produit fondant à 180°C. Ce produit est dissous dans 375 cm3 d'acétonitrile bouillant. La solution obtenue est additionnée de 0,5 g de noir décolorant et filtrée à chaud. Le filtrat est refroidi à une température voisine de 4°C pendant 16 heures. Les cristaux apparus sont séparés par filtration, lavés 2 fois par 50 cm3 au total d'acétonitrile refroidi à une température voisine de 4°C, 3 fois par 150 cm3 au total d'oxyde d'isopropyle et séchés sous pression réduite (20 mm de mercure ; 2,7 kPa) à une température voisine dè 20°C en présence de potasse en pastilles. On obtient ainsi 7,9 g de N-cyclopropyl (pyridyl-3)-5 1H,3H-pyrrolo [1,2-c] thiazolecarboxamide-7 sous forme de cristaux crême fondant à 184°C.

Le chlorhydrate de chloroformyl-7 (pyridyl-3)-5 1H,3H-pyrrolo[1,2-c] thiazole est préparé comme à l'exemple 1.

EXEMPLE 8 -

Une suspension de 12 g de chlorhydrate de chloroformyl-7 (pyridyl-3)-5 1H,3H-pyrrolo[1,2-c] thiazole dans 200 cm3 de chlorure de méthylène est additionnée en 20 minutes à une température comprise entre 21°C et 30°C d'une solution de 6,2 g d'amino-1 adamantane et de 8,1 g de triéthylamine dans 50 cm3 de chlorure de méthylène. La solution obtenue est agitée à une température voisine de 20°C pendant 16 heures puis est additionnée de 250 cm3 de chlorure de méthylène et de 250 cm3 d'eau distillée. La phase organique est séparée par décantation, lavée 3 fois par 750 cm3 au total d'eau distillée, séchée sur du sulfate de magnésium anhydre,

additionnée de 0,5 g de noir décolorant, filtrée et concentrée à sec sous pression réduite (20 mm de mercure ; 2,7 kPa) à une température voisine de 50°C. On obtient ainsi 11,9 g de produit. Ce produit est dissous dans 350 cm3 d'isopropanol bouillant. La solution obtenue est additionnée de 0,5 g de noir décolorant et filtrée à chaud. Le filtrat est refroidi à une température voisine de 4°C pendant 2 heures. Les cristaux apparus sont séparés par filtration, lavés 3 fois par 30 cm3 au total d'isopropanol refroidi à une température voisine de 4°C, 3 fois par 150 cm3 au total d'oxyde d'isopropyle et séchés sous prèssion réduite (20 mm de mercure ; 2,7 kPa) à une température voisine de 20°C en présence de potasse en pastilles. On obtient ainsi 4,1 g de N-(adamantyl-1) (pyridyl-3)-5 1H,3H-pyrrolo-[1,2-c] thiazolecarboxamide-7 sous forme de cristaux beiges fondant à 214°C.

Le chlorhydrate de chloroformyl-7 (pyridyl-3)-5 1H,3H-[1,2-c] thiazole est préparé comme à l'exemple 1.

EXEMPLE 9

Une suspension de 14,5 g de chlorhydrate de chloroformyl-7 (pyridyl-3)-5 1H,3H-pyrrolo[1,2-c] thiazole dans 240 cm3 de chlorure de méthylène est additionnée en 15 minutes à une température comprise entre 19°C et 32°C d'une solution de 8,2 g d'allylamine dans 60 cm3 de chlorure de méthylène. La solution obtenue est agitée à une température voisine de 20°C pendant 16 heures puis est additionnée de 300 cm3 de chlorure de méthylène et de 300 cm3 d'eau distillée. La phase organique est séparée par décantation, lavée 3 fois par 900 cm3 au total d'eau distillée, séchée sur du sulfate de magnésium anhydre, additionnée de 0,5 g de noir décolorant, filtrée et concentrée à sec sous pression réduite (20 mm de mercure ; 2,7 kPa) à une température voisine de 50°C. On obtient ainsi 11 g de produit fondant à 130°C. Ce produit est dissous dans 140 cm3 d'acétonitrile bouillant. La solution obtenue est additionnée de 0,5 g de noir décolorant et filtrée à chaud. Le filtrat est refroidi à une température voisine de 20°C pendant 2 heures. Les cristaux apparus

sont séparés par filtration, lavés 2 fois par 50 cm3 au total d'acétonitrile, 3 fois par 150 cm3 au total d'oxyde d'isopropyle et séchés sous pression réduite (20 mm de mercure ; 2,7 kPa) à une température voisine de 20°C en présence de potasse en pastilles. On obtient ainsi 6,7 g de N-allyl (pyridyl-3)-5 1H,3H-pyrrolo[1,2-c] thiazolecarboxamide-7 sous forme de cristaux beiges fondant à 132°C.

Le chlorhydrate de chloroformyl-7 (pyridyl-3)-5 1H,3H-pyrrolo[1,2-c] thiazole est préparé comme à l'exemple 1.

EXEMPLE 10 -

Une suspension de 15 g de chlorhydrate de chloroformyl-7 (pyridyl-3)-5 1H,3H-pyrrolo[1,2-c] thiazole dans 300 cm3 de chlorure de méthylène est additionnée en 30 minutes à une température comprise entre 26°C et 33°C d'une solution de 28,2 g de N-phényl-pipérazine dans 150 cm3 de chlorure de méthylène. La suspension obtenue est agitée à une température voisine de 20°C pendant 16 heures puis est additionnée de 300 cm3 de chlorure de méthylène et de 250 cm3 d'eau distillée. La phase organique est séparée par décantation, lavée 2 fois par 500 cm3 au total d'eau distillée, séchée sur du sulfate de magnésium anhydre, additionnée de 0,5 g de noir décolorant, filtrée et concentrée à sec sous pression réduite (20 mm de mercure ; 2,7 kPa) à une température voisine de 50°C. On obtient ainsi 22 g de produit. Ce produit est dissous dans 180 cm3 d'acétonitrile bouillant. La solution obtenue est additionnée de 0,5 g de noir décolorant et filtrée à chaud. Le filtrat est refroidi à une température voisine de 4°C pendant 2 heures. Les cristaux apparus sont séparés par filtration, lavés 2 fois par 50 cm3 au total d'acétonitrile et 3 fois par 75 cm3 au total d'éther diéthylique et séchés sous pression réduite (20 mm de mercure ; 2,7 kPa) à une température voisine de 20°C en présence de potasse en pastilles. On obtient ainsi 13,3 g de [(phényl-4 pipérazinyl-1) carbonyl]-7 (pyridyl-3)-5 1H,3H-pyrrolo[1,2-c] thiazole sous forme de cristaux crème fondant à 160°C.

Le chlorhydrate de chloroformyl-7 (pyridyl-3)-5 1H,3H-pyrrolo[1,2-c] thiazole est préparé comme à l'exemple 1.

EXEMPLE 11 -

Une solution d'éthoxymagnésien du malonate de diéthyle dans 65 cm3 d'un mélange d'éther diéthylique et d'éthanol (3-1 en volumes), préparée à partir de 1,34 g de magnésium et de 8,8 g de malonate de diéthyle, est additionnée à une température voisine de 20°C de 5,1 g de triéthylamine puis la suspension obtenue est additionnée en 5 minutes à une température voisine de 25°C de 15 g de chlorhydrate de chloroformyl-7 (pyridyl-3)-5 1H,3H-pyrrolo [1,2-c] thiazole. Au cours de cette période, le mélange réactionnel devient limpide et homogène avant de précipiter. La suspension obtenue est agitée à une température voisine de 20°C pendant 16 heures puis refroidie à une température voisine de 4°C et additionnée de 30 cm3 d'une solution aqueuse d'acide chlorhydrique 2N. Après passage du mélange par une phase limpide, on observe une précipitation. Les cristaux apparus sont séparés par filtration, lavés 3 fois par 90 cm3 au total d'eau distillée et séchés sous pression réduite (20 mm de mercure; 2,7 kPa) à une température voisine de 20°C. On obtient ainsi 13,5 g de produit. Le filtrat et les liqueurs de lavage sont réunis et extraits 3 fois par 240 cm3 au total d'acétate d'éthyle. Les extraits organiques sont réunis, séchés sur du sulfate de magnésium anhydre, additionnés de 0,5 g de noir décolorant, filtrés et concentrés à sec sous pression réduite (20 mm de mercure; 2,7 kPa) à une température voisine de 50°C. On obtient ainsi 6,1 g de produit. Ce produit est réuni aux 13,5 g de produit précédemment obtenus et est dissous dans un mélange de 25 cm3 d'acide acétique, 15 cm3 d'eau distillée et 3 cm3 d'acide sulfurique concentré. La solution obtenue est chauffée à l'ébullition pendant 3 heures puis refroidie à une température voisine de 20°C, diluée par 120 cm3 d'eau distillée, additionnée de 0,5 g de noir décolorant et filtrée. Le filtrat est amené à un pH voisin de 10 par addition de 17 cm3 d'une solution aqueuse de soude 10N. Les cristaux apparus sont séparés par filtration, lavés 3 fois par 90 cm3 au total de soude 10N et séchés sous pression réduite (20 mm de mercure; 2,7 kPa) à une température voisine de 20°C en présence de potasse en pastilles. On obtient ainsi 9,4 g de produit fondant à 155°C. Ce produit est dissous dans 130 cm3 d'éthanol bouillant. La solution obtenue est additionnée de 0,5 g de noir décolorant et filtrée à chaud. Le filtrat est refroidi à une température voisine de 4°C pendant 48 heures. Les cristaux apparus sont séparés par filtration, lavés 2 fois par 20 cm3 au total d'éthanol, 3 fois par 30 cm3 au total d'éther diéthylique et séchés sous pression réduite (20 mm de mercure; 2,7 kPa) à une température voisine de 20°C en présence de potasse en pastilles. On obtient ainsi 7, 2 g d'acétyl-7 (pyridyl-3)-5 1H,3H-pyrrrolo [1,2-c] thiazole sous forme de cristaux beige rosé fondant à 159°C.

Le chlorhydrate de chloroformyl-7 (pyridyl-3)-5 1H,3H-pyrrolo [1,2-c] thiazole est préparé comme à l'exemple 1.

L'éthoxymagnésien du malonate de diéthyle est préparé selon G.A. REYNOLDS et C.R. HAUSER, Org. Synth., Coll. Vol. 4, 708 (1963).

EXEMPLE 12 -

Une solution de bromure de phénylmagnésium dans 390 cm3 de tétrahydrofuranne anhydre (préparée à partir de 56,5 g de bromobenzène et de 8,8 g de magnésium) est additionnée en 5 minutes à une température voisine de 40°C d'une solution de 22,7 g de (pyridyl-3)-5 1H,3H-pyrrolo [1,2-c] thiazolecarbonitrile-7 dans 300 cm3 de tétrahydrofuranne anhydre et la suspension obtenue est chauffée à une température voisine de 66°C pendant 3 heures puis additionnée, à une température voisine de 10°C, de 495 cm3 d'eau distillée puis de 100 cm3 d'une solution aqueuse d'acide sulfurique 12N. La suspension obtenue est agitée à une température voisine de 20°C pendant 16 heures puis chauffée à l'ébullition pendant 7 heures et 30 minutes. La solution obtenue est refroidie à une température voisine de 20°C et la suspension obtenue est amenée à un pH voisin de 10 à une température voisine de 20°C par addition de 250 cm3 d'une solution

aqueuse de soude 5N. Après 1 heure d'agitation à une température voisine de 20°C, la suspension est filtrée et la phase organique est séparée par décantation. La phase aqueuse est extraite 3 fois par 750 cm3 au total d'acétate d'éthyle et les extraits organiques sont réunis, lavés 4 fois par 1000 cm3 au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 50°C. On obtient ainsi 25,2 g de produit fondant à 135°C. Ce produit est chromatographié sur une colonne de 4 cm de diamètre contenant 250 g de silice (0,063 - 0,2 mm) en éluant par des mélanges de cyclohexane et d'acétate d'éthyle et en recueillant des fractions de 250 cm3. Les deux premières fractions provenant de l'élution par un mélange de cyclohexane et d'acétate d'éthyle (50-50 en volumes) sont éliminées. Les trois fractions suivantes provenant de l'élution par un mélange de cyclohexane et d'acétate d'éthyle (50-50 en volumes ) et les trois fractions suivantes provenant de l'élution par de l'acétate d'éthyle pur sont réunies et concentrées à sec sous pression réduite (20 mm de mercure; 2,7 kPa) à une température voisine de 50°C. On obtient ainsi 17,4 g de produit. Ce produit est dissous dans 150 cm3 d'acétonitrile bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 50 cm3 au total d'acétonitrile refroidi à une température voisine de 4°C et 3 fois par 75 cm3 au total d'éther diéthylique et séchés sous pression réduite (20 mm de mercure; 2,7 kPa) à une température voisine de 20°C en présence de potasse en pastilles. On obtient ainsi 11,2 g de benzoyl-7 (pyridyl-3)-5 1H,3H-pyrrolo [1,2-c] thiazole sous forme de cristaux jaunes fondant à 152°C.

Le (pyridyl-3)-5 1H,3H-pyrrolo [1,2-c] thiazolecarbonitrile est préparé comme à l'exemple 1.

EXEMPLE 13 -

A une suspension de 12 g de chlorhydrate de chloroformyl-7 (pyridyl-3)-5 1H,3H,pyrrolo [1,2-c] thiazole dans 200 cm3 de chlorure de méthylène, on ajoute en 15 minutes à une température comprise entre 24°C et 29°C une solution de 11,2 g d'aniline dans 50 cm3 de chlorure de méthylène. La suspension obtenue est agitée à une température voisine de 20°C pendant 16 heures. On ajoute alors à la suspension 300 cm3 de chlorure de méthylène et 200 cm3 d'eau distillée. L'insoluble est séparé par filtration, lavé 3 fois par 75 cm3 au total de chlorure de méthylène et 3 fois par 75 cm3 au total d'eau distillée. Les liqueurs-mères de filtration et de lavage sont réunies; la phase organique est décantée, lavée 2 fois par 400 cm3 au total d'eau distillée, 1 fois par 200 cm3 d'une solution aqueuse de soude 2N puis 2 fois par 400 cm3 au total d'eau distillée, séchée sur du sulfate de magnésium anhydre,additionnée de 0,5 g de noir décolorant et filtrée. On obtient ainsi 625 cm3 d'une solution chlorométhylénique brute de produit. L'insoluble précédemment obtenu est repris par 200 cm3 d'une solution aqueuse de soude 5N. L'huile obtenue est extraite 3 fois par 750 cm3 au total de chlorure de méthylène. Les extraits organiques sont réunis, lavés 3 fois par 450 cm3 au total d'eau distillée, séchés sur du sulfate de magnésium anhydre, additionnés de 0,5 g de noir décolorant et filtrés. La solution chlorométhylénique obtenue est réunie aux 625 cm3 de solution chlorométhylénique précédemment obtenus et le solvant est évaporé sous pression réduite (20 mm de mercure; 2,7 kPa) à une température voisine de 60°C. On obtient ainsi 15,4 g de produit brut. Ce produit est dissous dans 210 cm3 d'acétonitrile bouillant. La solution obtenue est additionnée de 0,5 g de noir décolorant et filtrée à chaud. Le filtrat est refroidi à une température voisine de 4°C pendant 2 heures. Les cristaux apparus sont séparés par filtration, lavés 3 fois par 30 cm3 au total d'acétonitrile refroidi à une température voisine de 4°C et 3 fois par 75 cm3 au total d'oxyde d'isopropyle puis séchés sous pression réduite

(20 mm de mercure; 2,7 kPa) à une température voisine de 20°C en présence de potasse en pastilles. On obtient ainsi 7,4 g de N-phényl (pyridyl-3)-5 1H,3H-pyrrolo [1,2-c] thiazolecarboxamide-7 sous forme de cristaux crème fondant à 188°C.

Le chlorhydrate de chloroformyl-7 (pyridyl-3)-5 1H,3H-pyrrolo [1,2-c] thiazole est préparé comme à l'exemple 1.

EXEMPLE 14

A une solution de 24,5 g de (pyridyl-3)-5 1H,3H-pyrrolo [1,2-c] thiazolecarboxamide-7 dans 330 cm3 d'acide acétique, on ajoute en 20 minutes à une température voisine de 20°C une solution de 17,1 g de brome dans 70 cm3 d'acide acétique. La suspension obtenue est agitée à une température voisine de 20°C pendant 3 heures. Les cristaux sont alors séparés par filtration, lavés 3 fois par 150 cm3 au total d'acide acétique, 3 fois par 150 cm3 au total d'acétone et 3 fois par 150 cm3 au total d'éther diéthylique puis séchés sous pression réduite (20 mm de mercure; 2,7 kPa) à une température voisine de 20°C en présence de potasse en pastilles. Les 60 g de produit obtenus sont mis en suspension dans 1300 cm3 d'une solution aqueuse de soude 6,15N. Les cristaux obtenus sont séparés par filtration, lavés 4 fois par 1000 cm3 au total d'eau distillée et séchés sous pression réduite (20 mm de mercure; 2,7 kPa) à une température voisine de 20°C. Les 28,5 g de produit ainsi obtenus sont chromatographiés sur une colonne de 4,2 cm de diamètre contenant 300 g de silice (0,063 - 0,2 mm) en éluant par des mélanges d'acétate d'éthyle et de méthanol et en recueillant des fractions de 1000 cm3. Les 3 premières fractions provenant de l'élution par de l'acétate d'éthyle pur sont réunies et concentrées à sec sous pression réduite (20 mm de mercure; 2,7 kPa) à une température voisine de 60°C. On obtient ainsi 0,9 g de produit. Les 7 fractions suivantes provenant de l'élution par un mélange d'acétate d'éthyle et de méthanol (95-5 en volumes) et les 3 fractions suivantes provenant de l'élution par un mélange d'acétate d'éthyle et de méthanol (90-10 en volumes) sont réunies et concentrées à sec sous

pression réduite (20 mm de mercure; 2,7 kPa) à une température voisine de 60°C. On obtient ainsi 18,1 g de produit qui sont mis en suspension dans 400 cm3 d'un mélange de chlorure de méthylène et de méthanol (96-4 en volumes). La suspension obtenue est filtrée et le filtrat est concentré à sec sous pression réduite (20 mm de mercure; 2,7 kPa) à une température voisine de 60°C. On obtient ainsi 10,1 g de produit. Ce produit est chromatographié sur une colonne de 6 cm de diamètre contenant 480 g de silice (0,04 - 0,063 mm). On élue par un mélange de chlorure de méthylène et de méthanol (96-4 en volumes) sous une pression de 0,5 bar (51 kPa) en recueillant des fractions de 100 cm3. Les 4 premières fractions sont éliminées, les 13 fractions suivantes sont réunies et concentrées à sec sous pression réduite (20 mm de mercure; 2,7 kPa) à une température voisine de 60°C. On obtient ainsi 6,4 g de produit fondant à 230°C. Ce produit réuni aux 0,9 g de produit obtenus précédemment, est dissous dans 200 cm3 de butanol-1 bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 50 cm3 au total de butanol-1, 2 fois par 50 cm3 au total d'éthanol et 2 fois par 50 cm3 au total d'éther diéthylique puis séchés sous pression réduite (20 mm de mercure; 2,7 kPa) à une température voisine de 20°C en présence de potasse en pastilles. On otient ainsi 5,6 g de bromo-6 (pyridyl-3)-5 1H,3H-pyrrolo [1,2-c] thiazolecarboxamide-7 sous forme de cristaux jaune pâle fondant à 232°C.

Le (pyridyl-3)-5 1H,3H-pyrrolo [1,2-c] thiazolecarboxamide-7 peut-être préparé de la manière suivante :

Une suspension de 11,35g de (pyridyl-3)-5 1H,3H-pyrrolo [1,2-c] thiazolecarbonitrile-7 et de 14 g de potasse en poudre dans 100 cm3 d'alcool tert-butylique est chauffée à 85°C pendant 1 heure. Après 16 heures d'agitation à une température voisine de 20°C, le mélange réactionnel est versé sur 2 litres d'eau distillée. La suspension est agitée à une température voisine de 20°C pendant 15 minutes puis les cristaux apparus sont séparés par filtration, lavés 8 fois par 1200 cm3 au total d'eau distillée puis 3 fois par 150 cm3

au total d'éthanol et séchés sous pression réduite (20 mm de mercure; 2,7 kPa) à une température voisine de 20°C en présence de potasse en pastilles. On obtient ainsi 10,5 g de produit brut qui sont réunis à 3,9 g de produit préparé de la même façon dans une opération antérieure et dissous dans 850 cm3 d'éthanol bouillant. La solution est additionnée de 0,5 g de noir décolorant et filtrée à chaud. Le filtrat est refroidi à une température voisine de 4°C pendant 3 jours. Les cristaux apparus sont séparés par filtration, lavés 3 fois par 30 cm3 au total d'éthanol refroidi à une température voisine de 4°C et séchés sous pression réduite (20 mm de mercure; 2,7 kPa) à une température voisine de 20°C en présence de potasse en pastilles. On obtient ainsi 11,3 g de (pyridyl-3)-5 1H,3H-pyrrolo [1,2-c] thiazolecarboxamide-7 sous forme de cristaux crème fondant à 215°C.

EXEMPLE 15 -

Une suspension de 3,6 g de potasse en poudre et de 2,6 g de méthyl-6 (pyridyl-3)-5 1H,3H-pyrrolo [1,2-c] thiazolecarbonitrile-7 dans 75 cm3 d'alcool tert-butylique est chauffée à une température voisine de 81°C pendant 10 heures. La suspension est versée sur 500 cm3 d'eau distillée et la solution obtenue est agitée à une température voisine de 4°C pendant 3 heures. Les cristaux apparus sont séparés par filtration, lavés 5 fois par 125 cm3 au total d'eau distillée et séchés sous pression réduite (20 mm de mercure; 2,7 kPa) à une température voisine de 20°C en présence de potasse en pastilles. On obtient ainsi 2,3 g de produit fondant à 212°C. Ce produit est dissous dans 150 cm3 d'éthanol bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 50 cm3 au total d'éthanol et 3 fois par 75 cm3 au total d'éther diéthylique et séchés sous pression réduite (20 mm de mercure; 2,7 kPa) à une température voisine de 20°C en présence de potasse en pastilles. On obtient ainsi 1,6 g de méthyl-6

(pyridyl-3)-5 1H,3H-pyrrolo [1,2-c] thiazolecarboxamide-7 sous forme de cristaux blancs fondant à 222°C.

Le méthyl-6 (pyridyl-3)-5 1H,3H-pyrrolo [1,2-c] thiazolecarbonitrile-7 peut être préparé de la façon suivante :

Une suspension de 19 g d'acide N-nicotinoylthiazolidinecarboxylique-4 et de 58,4 g de bromo-2 butène-2 nitrile dans 120 cm3 d'anhydride acétique est chauffée à une température voisine de 100°C pendant 1 heure. La solution obtenue est concentrée à sec sous pression réduite (20 mm de mercure; 2,7 kPa) à une température voisine de 80°C. Le résidu est mis en suspension dans 150 cm3 d'eau distillée. La suspension est additionnée de 150 cm3 d'une solution aqueuse de soude 10N et agitée à une température voisine de 20°C pendant 30 minutes. On ajoute alors 300 cm3 de chlorure de méthylène; la phase organique est séparée par décantation et la phase aqueuse est extraite 2 fois par 600 cm3 au total de chlorure de méthylène. Les extraits organiques sont réunis, lavés 3 fois par 300 cm3 au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 60°C. On obtient ainsi 2,6 g de produit brut. Ce produit est chromatographié sur une colonne de 4 cm de diamètre contenant 320 g de silice (0,04 - 0,063 mm). On élue par un mélange d'acétate d'éthyle et de cyclohexane (70-30 en volumes) sous une pression de 0,5 bar (51 kPa) et en recueillant des fractions de 50 cm3. Les 9 premières fractions sont éliminées, les 8 fractions suivantes sont réunies et concentrées à sec sous pression réduite (20 mm de mercure; 2,7 kPa) à une température voisine de 60°C. On obtient ainsi 1,1 g de produit. Ce produit est réuni avec 4 g de produit préparé de la même façon dans des opérations antérieures et est dissous dans 75 cm3 d'acétonitrile bouillant. La solution obtenue est filtrée à chaud. Le filtrat est refroidi à une température voisine de 20°C. Les cristaux apparus sont séparés par filtration, lavés 2 fois par 20 cm3 au total d'acétonitrile et 3 fois par 75 cm3 au total d'éther diéthylique et séchés sous pression réduite

20 mm de mercure; 2,7 kPa) à une température voisine de 20°C en présence de potasse en pastilles. On obtient ainsi 2,6 g de méthyl-6 (pyridyl-3)-5 1H,3H-pyrrolo [1,2-c] thiazolecarbonitrile-7 sous forme de cristaux crème fondant à 190°C.

L'acide N-nicotinoyl thiazolidinecarboxylique-4 est préparé comme à l'exemple 1.

Le bromo-2 butène-2 nitrile peut être préparé selon L. LECLERCQ et A. BRUYLANTS, Bull. Soc. chim. belges, 58, 5 (1949).

EXEMPLE 16

A une suspension de 15 g de chlorhydrate de chloroformyl-7 (pyridyl-3)-5 1H,3H-pyrrolo [1,2-c] thiazole dans 250 cm3 de chlorure de méthylène, on ajoute en 30 minutes à une température comprise entre 26 et 34°C, une solution de 6,4 g de chloro-3 aniline et de 10,1 g de triéthylamine dans 100 cm3 de chlorure de méthylène. La solution obtenue est agitée à une température voisine de 20°C pendant 16 heures. Un produit précipite. Les cristaux apparus sont séparés par filtration, lavés 4 fois par 400 cm3 au total de chlorure de méthylène et séchés sous pression réduite (20 mm de mercure; 2,7 kPa) à une température voisine de 20°C en présence de potasse en pastilles. On obtient ainsi 9 g de produit fondant à 200°C. Les filtrats précédents sont réunis, lavés 4 fois par 800 cm3 au total d'eau distillée puis séchés sur du sulfate de magnésium anhydre et concentrés à sec sous pression réduite (20 mm de mercure; 2,7 kPa) à une température voisine de 60°C. On obtient ainsi 7 g de produit fondant à 198°C que l'on réunit aux 9 g de produit précédemment obtenus et que l'on dissout dans 250 cm3 de butanol-1 bouillant. La solution obtenue est additionnée de 0,5 g de noir décolorant et filtrée à chaud; le filtrat est refroidi à une température voisine de 4°C pendant 16 heures. Les cristaux apparus sont séparés par filtration, lavés 3 fois par 75 cm3 au total de butanol-1, 3 fois par 75 cm3 au total d'éthanol, et 3 fois par 150 cm3 au total d'éther diéthylique puis séchés sous pression réduite (20 mm de mercure; 2,7 kPa) à une température voisine de 20°C en présence de potasse en pastilles.

On obtient ainsi 11,9 g de N-(chloro-3 phényl) (pyridyl-3)-5 1H,3H-pyrrolo [1,2-c] thiazolecarboxamide-7 sous forme de cristaux crème fondant à 206°C.

Le chlorhydrate de chloroformyl-7 (pyridyl-3)-5 1H,3H-pyrrolo [1,2-c] thiazole est préparé comme à l'exemple 1.

EXEMPLE 17

Une suspension de 14,1 g de chlorhydrate de chloroformyl-7 (pyridyl-3)-5 dihydro-2,3 1H-pyrrolizine dans 250 cm3 de chlorure de méthylène est saturée par un courant de méthylamine anhydre en maintenant la température du mélange réactionnel voisine de 25°C pendant 7 heures. La solution obtenue est agitée à une température voisine de 20°C pendant 16 heures puis est additionnée de 250 cm3 de chlorure de méthylène et de 250 cm3 d'eau distillée. La phase organique est séparée par décantation, lavée 3 fois par 750 cm3 au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 12,2 g de produit brut fondant à 184°C. Ce produit est dissous dans 150 cm3 d'acétonitrile bouillant. La solution obtenue est additionnée de 0,5 g de noir décolorant et filtrée à chaud. Le filtrat est refroidi à une température voisine de 4°C. pendant 1 heure. Les cristaux apparus sont séparés par filtration, lavés 2 fois par 20 cm3 au total d'acétonitrile refroidi à une température voisine de 4°C et 3 fois par 75 cm3 au total d'éther diéthylique puis séchés sous pression réduite (20 mm de mercure; 2,7 kPa) à une température voisine de 20°C en présence de potasse en pastilles. On obtient ainsi 7,1 g de N-méthyl (pyridyl-3)-5 dihydro-2,3 1H-pyrrolizinecarboxamide-7 sous forme de cristaux crème fondant à 187°C.

Le chlorhydrate de chloroformyl-7 (pyridyl-3)-5 dihydro-2,3 1H-pyrrolizine peut être préparé de la façon suivante :

Une suspension de 11,4 g d'acide (pyridyl-3)-5 dihydro-2,3 1H-pyrrolizinecarboxylique-7 dans un mélange de 17,4 cm3 de chlorure de thionyle, de 0,05 cm3 de diméthylformamide et de 150 cm3 de dichloro-1,2 éthane est chauffée à une température voisine de 80°C pendant 3 heures. Le mélange réactionnel est refroidi à une température voisine de 20°C et concentré à sec sous pression réduite (20 mm de mercure; 2,7 kPa) à une température voisine de 60°C. Le résidu obtenu est mis en suspension dans 250 cm3 de cyclohexane et le solvant est évaporé sous pression réduite (20 mm de mercure; 2,7 kPa) à une température voisine de 60°C. La même opération est répétée 2 fois. On obtient ainsi 14,1 g de chlorhydrate de chloroformyl-7 (pyridyl-3)-5 dihydro-2,3 1H-pyrrolizine sous forme de cristaux jaunes fondant à 230°C.

L'acide (pyridyl-3)-5 dihydro-2,3 1H-pyrrolizinecarboxylique-7 peut être préparé de la façon suivante :

Une suspension de 41,9 g de (pyridyl-3)-5 dihydro-2,3 1H-pyrrolizinecarbonitrile-7 et de 39,6 g de potasse en poudre dans 400 cm3 d'éthylèneglycol est chauffée à une température voisine de 160°C. La solution obtenue est maintenue à une température voisine de 160°C pendant 3 heures. Le solvant est évaporé sous pression réduite (20 mm de mercure; 2,7 kPa) à une température voisine de 110°C. Le résidu obtenu est dissous dans 1100 cm3 d'eau distillée et la solution est amenée à un pH voisin de 5 par addition de 300 cm3 d'une solution aqueuse d'acide chlorhydrique 2N. La suspension obtenue est agitée à une température voisine de 20°C pendant 1 heure. Les cristaux sont séparés par filtration, lavés 2 fois par 500 cm3 au total d'eau distillée et séchés à l'air puis sous pression réduite (20 mm de mercure; 2,7 kPa) à une température voisine de 20°C en présence de potasse en pastilles. On obtient ainsi 58 g de produit brut hydraté fondant à 220°C. Le produit est repris par 1500 cm3 d'éthanol bouillant et la suspension obtenue est filtrée à chaud. Le filtrat est refroidi à une température voisine de 4°C pendant 16 heures. Les cristaux apparus sont séparés par filtration, lavés 3 fois par 300 cm3 au total d'éthanol et 3 fois par 300 cm3 au total d'éther diéthylique puis séchés sous pression réduite (20 mm de mercure; 2,7 kPa) à une température voisine de de 20°C en

présence de potasse en pastilles. On obtient ainsi 29,4 g d'acide (pyridyl-3)-5 dihydro-2,3 1H-pyrrolizinecarboxylique-7 sous forme de cristaux jaune pâle fondant à 240°C.

Le (pyridyl-3)-5 dihydro-2,3 1H-pyrrolizinecarbonitrile peut être préparé comme décrit dans la demande de brevet européen publiée sous le n° 0 118 321.

EXEMPLE 18

Une suspension de 16,3 g de chlorhydrate de chloroformyl-1 (pyridyl-3)-3 tétrahydro-5,6,7,8 indolizine dans 275 cm3 de chlorure de méthylène est saturée par un courant de méthylamine anhydre en maintenant la température du mélange réactionnel voisine de 25°C pendant 7 heures. La solution obtenue est agitée à une température voisine de 20°C pendant 16 heures puis est additionnée de 500 cm3 de chlorure de méthylène et de 300 cm3 d'eau dstillée. La phase organique est séparée par décantation, lavée 4 fois par 1200 cm3 au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 13,9 g de produit fondant à 168°C. Ce produit est dissous dans 150 cm3 d'acétonitrile bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 3 fois par 30 cm3 au total d'acétonitrile refroidi à une température voisine de 4°C et 3 fois par 75 cm3 au total d'éther diéthylique puis séchés sous pression réduite (20 mm de mercure; 2,7 kPa) à une température voisine de 20°C en présence de potasse en pastilles. On obtient ainsi 8,3 g de N-méthyl (pyridyl-3)-3 tétrahydro-5,6,7,8 indolizinecarboxamide-1 sous forme de cristaux jaune clair fondant à 170°C.

Le chlorhydrate de chloroformyl-1 (pyridyl-3)-3 tétrahydro-5,6,7,8 indolizine peut être préparé de la façon suivante :

Une solution de 13,3 g d'acide (pyridyl-3)-3 tétrahydro-5,6,7,8 indolizinecarboxylique-1 dans un mélange de 19,2 cm3 de chlorure de thionyle, 0,05 cm3 de diméthylformamide et de 160 cm3 de dichloro-1,2 éthane est chauffée à une température voisine de 70°C. La suspension obtenue est maintenue à une température voisine de 70°C pendant 3 heures. Le mélange réactionnel est refroidi à une température voisine de 20°C et concentré à sec sous pression réduite (20 mm de mercure; 2,7 kPa) à une température voisine de 60°C. Le résidu est mis en suspension dans 300 cm3 de cyclohexane et le solvant est évaporé sous pression réduite (20 mm de mercure; 2,7 kPa) à une température voisine de 60°C. La même opération est répétée deux fois. On obtient ainsi 16,3 g de chlorhydrate de chloroformyl-1 (pyridyl-3)-3 tétrahydro-5,6,7,8 indolizine sous forme de cristaux vert clair fondant à 205°C.

L'acide (pyridyl-3)-3 tétrahydro-5,6,7,8 indolizinecarboxylique-1 peut être préparé de la façon suivante :

Une solution de 15,6 g de (pyridyl-3)-3 tétrahydro-5,6,7,8 indolizinecarbonitrile-1 et de 15,8 g de potasse en poudre dans 150 cm3 d'éthylèneglycol est chauffée à une température voisine de 156°C pendant 11 heures et 30 minutes. Après 16 heures d'agitation à une température voisine de 20°C, le solvant est évaporé sous pression réduite (2 mm de mercure; 0,27 kPa) à une température voisine de 100°C. Le résidu obtenu est dissous dans 250 cm3 d'eau distillée. La solution obtenue est additionnée de 0,5 g de noir décolorant et filtrée. Le filtrat est amené à un pH voisin de 5 à une température voisine de 25°C par addition de 50 cm3 d'une solution aqueuse d'acide chlorhydrique 5N. La suspension obtenue est agitée à une température voisine de 20°C pendant 1 heure. Les cristaux sont séparés par filtration, lavés 5 fois par 250 cm3 au total d'eau distillée puis séchés à l'air. On obtient ainsi 13,4 g d'acide (pyridyl-3)-3 tétrahydro-5,6,7,8 indolizinecarboxylique-1 sous forme de cristaux beiges fondant à 198°C.

Le (pyridyl-3)-3 tétrahydro-5,6,7,8 indolizinecarbonitrile-1 est préparé comme décrit dans la demande de brevet européen publiée sous le n° 0 118 321.

EXEMPLE 19

Une suspension de 27,3 g d'acide N-nicotinoyl tétrahydro-3,4,5,6 2H-thiazine-1,3 carboxylique-4 dans 250 cm3 d'anhydride acétique est chauffée à une température voisine de 95°C. A la solution obtenue, on ajoute en 15 minutes, une solution de 18 g de N-méthyl propiolamide dans 190 cm3 d'anhydride acétique en maintenant la température au voisinage de 95°C. On maintient cette température pendant encore 1 heure. On obtient alors une suspension. Cette suspension est refroidie à une température voisine de 4°C pendant 1 heure. Les cristaux apparus sont séparés par filtration, lavés 2 fois par 20 cm3 au total d'anhydride acétique refroidi à une température voisine de 4°C et 3 fois par 60 cm3 au total d'éther diéthylique puis séchés sous pression réduite (20 mm de mercure; 2,7 kPa) à une température voisine de 20°C en présence de potasse en pastilles. On obtient ainsi 12 g de produit fondant à 245°C. Ce produit est dissous dans 200 cm3 de butanol-1 bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 20 cm3 au total de butanol-1 refroidi à une température voisine de 4°C et 4 fois par 200 cm3 au total d'éther diéthylique, puis séchés sous pression réduite (20 mm de mercure; 2,7 kPa) à une température voisine de 20°C en présence de potasse en pastilles. On obtient ainsi 10,5 g de N-méthyl (pyridyl-3)-6 dihydro-1,2 4H-pyrrolo [1,2-c] thiazine-1,3 carboxamide-8 sous forme de cristaux jaunes fondant à 248°C.

L'acide N-nicotinoyl tétrahydro-3,4,5,6 2H-thiazine-1,3 carboxylique-4 est préparé comme décrit dans la demande de brevet européen publiée sous le n° 0 118 321.

Le N-méthyl propiolamide est préparé selon W.D. CROW et N.J. LEONARD, J. Org. Chem., 30, 2660 (1965).

EXEMPLE 20

A une suspension de 15 g de chlorhydrate de chloroformyl-7 (pyridyl-3)-5 pyrrolo [1,2-c] thiazole dans 250 cm3 de chlorure de méthylène, on ajoute en 15 minutes à une température comprise entre 20 et 30°C une solution de 4,7 g d'amino-3 pyridine et de 10,2 g de triéthylamine dans 100 cm3 de chlorure de méthylène. La solution obtenue est agitée à une température voisine de 20°C pendant 16 heures. Un produit précipite. Les cristaux sont séparés par filtration, lavés 4 fois par 200 cm3 au total de chlorure de méthylène, 3 fois par 200 cm3 au total d'eau distillée, 2 fois par 200 cm3 au total d'une solution aqueuse de soude 2N, 4 fois par 200 cm3 au total d'eau distillée, 3 fois par 75 cm3 au total d'acétone et 3 fois par 75 cm3 au total d'éther diéthylique puis séchés sous pression réduite (20 mm de mercure; 2,7 kPa) à une température voisine de 20°C en présence de potasse en pastilles. On obtient ainsi 13,2 g de produit fondant à 260°C. Ce produit est dissous dans 110 cm3 de diméthylformamide bouillant. La solution obtenue est additionnée de 0,5 g de noir décolorant et filtrée à chaud. Le filtrat est refroidi à une température voisine de 4°C pendant 2 heures. Les cristaux apparus sont séparés par filtration, lavés 3 fois par 30 cm3 au total de diméthylformamide refroidi à une température voisine de 4°C, 3 fois par 75 cm3 au total d'éthanol et 3 fois par 75 cm3 au total d'éther diéthylique puis séchés sous pression réduite (20 mm de mercure; 2,7 kPa) à une température voisine de 20°C en présence de potasse en pastilles. On obtient ainsi 11,6 g de N-(pyridyl-3) (pyridyl-3)-5 1H,3H-pyrrolo [1,2-c] thiazolecarboxamide-7 sous forme de cristaux beiges fondant à 278°C.

Le chlorhydrate de chloroformyl-7 (pyridyl-3)-5 pyrrolo [1,2-c] thiazole est préparé comme à l'exemple 1.

EXEMPLE 21

A une suspension de 15 g de chlorhydrate de chloroformyl-7 (pyridyl-3)-5 1H,3H-pyrrolo [1,2-c] thiazole dans 250 cm3 de chlorure de méthylène, on ajoute en 15 minutes à une température comprise entre 20 et 30°C une solution de 5,5 g d'aminométhyl-3

pyridine et de 10,1 g de triéthylamine dans 100 cm3 de chlorure de méthylène. La solution obtenue est agitée à une température voisine de 20°C pendant 16 heures. Un produit précipite. Les cristaux apparus sont séparés par filtration, lavés 3 fois par 300 cm3 au total de chlorure de méthylène et 3 fois par 200 cm3 au total d'oxyde d'isopropyle puis séchés sous pression réduite (20 mm de mercure; 2,7 kPa) à une température voisine de 20°C en présence de potasse en pastilles. On obtient ainsi 11,3 g de produit brut fondant à 206°C. Ce produit est dissous dans 160 cm3 de butanol-1 bouillant. La solution obtenue est additionnnée de 0,5 g de noir décolorant et filtrée à chaud. Le filtrat est refroidi à une température voisine de 4°C pendant 2 heures. Les cristaux apparus sont séparés par filtration, lavés 4 fois par 100 cm3 au total de butanol-1 et 4 fois par 100 cm3 au total d'éther diéthylique puis séchés sous pression réduite (20 mm de mercure; 2,7 kPa) à une température voisine de 20°C en présence de potasse en pastilles. On obtient ainsi 9,9 g de N-(pyridyl-3 méthyl) (pyridyl-3)-5 1H,3H-pyrrolo [1,2-c] thiazolecarboxamide-7 sous forme de cristaux crème fondant à 210°C.

Le chlorhydrate de chloroformyl-7 (pyridyl-3)-5 1H,3H-pyrrolo [1,2-c] thiazole est préparé comme à l'exemple 1.

EXEMPLE 22

Une suspension de 6,5 g de [(pyridyl-3)-5 1H,3H-pyrrolo [1,2-c] thiazolyl-7]-2 acétonitrile et de 8,9 g de potasse en poudre dans 110 cm3 d'alcool tert-butylique est chauffée à une température voisine de 80°C pendant 15 minutes. La suspension est versée sur 1500 cm3 d'eau distillée et le mélange est extrait 4 fois par 1200 cm3 au total de chlorure de méthylène. Les extraits organiques sont réunis, lavés 3 fois par 750 cm3 au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 60°C. On obtient ainsi 4,9 g de produit fondant à 174°C. Ce produit est réuni à 0,6 g de produit identique préparé dans une autre opération antérieure , et le tout est dissous dans 150 cm3 d'éthanol bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 3 fois par 75 cm3 au total d'éthanol refroidi à une témpérature voisine de 4°C et 3 fois par 75 cm3 au total d'éther diéthylique puis séchés sous pression réduite (20 mm de mercure; 2,7 kPa) à une température voisine de 20°C en présence de potasse en pastilles. On obtient ainsi 3,4 g de [(pyridyl-3)-5 1H,3H-pyrrolo [1,2-c] thiazolyl-7]-2 acétamide sous forme de cristaux jaune pâle fondant à 176°C.

Le [(pyridyl-3)-5 1H,3H-pyrrolo [1,2-c] thiazolyl-7]-2 acétonitrile peut être préparé de la façon suivante :

A une solution de 8,7 g de tert-butylate de potassium dans 140 cm3 de diméthoxy-1,2 éthane refroidie à une température voisine de -30°C, on ajoute en 20 minutes, à une température voisine de -30°C, une solution de 7,5 g d'isocyanure de tosylméthyle dans 50 cm3 de diméthoxy-1,2 éthane. La solution obtenue est refroidie à une température voisine de -60°C puis additionnée en 30 minutes, à une température voisne de -60°C, d'une solution de 8 g de (pyridyl-3)-5 1H,3H-pyrrolo [1,2-c] thiazolecarboxaldéhyde-7 dans 70 cm3 de diméthoxy-1,2 éthane. La solution obtenue est amenée en 1 heure à une température voisine de 0°C puis maintenue à cette température pendant 1 heure et 20 minutes. On ajoute alors en 15 minutes, à une température comprise entre 3 et 7°C, 105 cm3 de méthanol. La solution obtenue est chauffée à une température voisine de 72°C pendant 1 heure et 15 minutes puis est agitée à une température voisine de 20°C pendant 16 heures. Les trois-quarts du solvant sont évaporés sous pression réduite (20 mm de mercure; 2,7 kPa) à une température voisine de 40°C. Le résidu est repris par un mélange de 350 cm3 d'eau distillée et de 250 cm3 d'acétate d'éthyle. La phase organique est séparée par décantation et la phase aqueuse est extraite 3 fois par 750 cm3 au total d'acétate d'éthyle. Les extraits organiques sont réunis, lavés 3 fois par 450 cm3 au total

d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 50°C. On obtient ainsi 7,6 g de produit brut. Ce produit est chromatographié sur une colonne de 2,7 cm de diamètre contenant 75 g de silice (0,063 - 0,2 mm) en éluant par des mélanges de cyclohexane et d'acétate d'éthyle et en recueillant des fractions de 200 cm3. Les deux premières fractions provenant de l'élution par un mélange de cyclohexane et d'acétate d'éthyle (50-50 en volumes) sont éliminées. Les trois fractions suivantes provenant de l'élution par un mélange de cyclohexane et d'acétate d'éthyle (50-50 en volumes) et la fraction suivante provenant de l'élution par de l'acétate d'éthyle pur sont réunies et concentrées à sec sous pression réduite (20 mm de mercure; 2,7 kPa) à une température voisine de 60°C. On obtient ainsi 6 g de [(pyridyl-3)-5 1H,3H-pyrrolo [1,2-c] thiazolyl-7]-2 acétonitrile sous forme de cristaux jaune clair fondant à 92°C.

Le (pyridyl-3)-5 1H,3H-pyrrolo [1,2-c] thiazolecarboxaldéhyde-7 est préparé comme décrit dans la demande de brevet européen publiée sous le numéro 0 118 321.

EXEMPLE 23

Une suspension de 6,5 g de potasse en poudre et de 4,6 g d'un mélange (dans la proportion 20-80) de cyano-6 et de cyano-7 (thiazolyl-5)-5 1H,3H-pyrrolo [1,2-c] thiazole dans 100 cm3 d'alcool tert-butylique est portée au reflux pendant 2 minutes dans un bain préchauffé à 100°C. La suspension résultante est versée dans 800 cm3 d'eau distillée et les cristaux obtenus sont séparés par filtration, lavés 6 fois par 300 cm3 au total d'eau distillée et 3 fois par 75 cm3 au total d'acétone puis séchés sous pression réduite (20 mm de mercure; 2,7 kPa) à une température voisine de 20°C en présence de potasse en pastilles. On obtient ainsi 1,8 g de produit fondant à 230°C. Le filtrat précédent est extrait 3 fois par 1000 cm3 au total de chlorure de méthylène et 3 fois par 1000 cm3 au total d'acétate d'éthyle. Les extraits organiques sont réunis, séchés sur du sulfate

de magnésium anhydre, filtrés et concentrés à sec sous pression réduite (20 mm de mercure; 2,7 kPa) à une température voisine de 60°C. On obtient ainsi 1 g de produit fondant à 215°C. Ce produit est réuni aux 1,8 g obtenus précédemment et à 1,2 g de produit identique provenant d'une autre opération antérieure. Les trois lots sont dissous dans 200 cm3 de butanol-1 bouillant. La solution obtenue est additionnée de 0,5 g de noir décolorant et filtrée à chaud. Le filtrat est refroidi à une température voisine de 4°C pendant 2 heures. Les cristaux apparus sont séparés par filtration, lavés 2 fois par 20 cm3 au total de butanol-1, 2 fois par 50 cm3 au total d'éthanol et 3 fois par 75 cm3 au total d'éther diéthylique puis séchés sous pression réduite (20 mm de mercure; 2,7 kPa) à une température voisine de 20°C en présence de potasse en pastilles. On obtient ainsi 2,1 g de (thiazolyl-5)-5 1H,3H-pyrrolo [1,2-c] thiazolecarboxamide-7 sous forme de cristaux orangés fondant à 236°C.

Le mélange (20-80) de cyano-6 et de cyano-7 (thiazolyl-5)-5 1H,3H-pyrrolo [1,2-c] thiazole peut être préparé de la façon suivante :

A une solution de 13,1 g d'acide thiazolidinecarboxylique-4 et de 9,8 g de triéthylamine dans 360 cm3 de chloroforme, on ajoute en 15 minutes à une température comprise entre 23 et 36°C, une solution de 14,1 g de chloroformyl-5 thiazole dans 120 cm3 de chloroforme. La solution obtenue est chauffée à une température voisine de 64°C pendant 2 heures et 15 minutes. Après agitation à une température voisine de 20°C pendant 16 heures, le solvant est évaporé sous pression réduite (20 mm de mercure; 2,7 kPa) à une température voisine de 60°C et le résidu est repris par 350 cm3 d'acétone. La suspension obtenue est filtrée et le filtrat est concentré à sec sous pression réduite (20 mm de mercure; 2,7 kPa) à une température voisine de 60°C. On obtient ainsi 26,4 g de produit que l'on dissout dans un mélange de 77 cm3 de chloro-2 acrylonitrile et de 96 cm3 d'anhydride acétique. La solution obtenue est chauffée à une température voisine de 90°C pendant 2 heures et 45 minutes, puis est agitée à une température voisine de 20°C pendant 16 heures.

Les cristaux apparus sont séparés par filtration, lavés 2 fois par 150 cm3 au total d'anhydride acétique et 3 fois par 225cm3 au total d'acétone puis séchés sous pression réduite (20mm de mercure; 2,7 kPa) à une température voisine de 20°C en présence de potasse en pastilles. On obtient ainsi 7,2 g de produit. Ce produit est mis en suspension dans 150 cm3 d'eau distillée. La suspension est additionnée de 150 cm3 d'une solution aqueuse de soude 10N. Les cristaux apparus sont séparés par filtration, lavés 4 fois par 200 cm3 au total d'eau distillée et séchés sous pression réduite (20 mm de mercure; 2,7 kPa) à une température voisine de 20°C en présence de potasse en pastilles. On obtient ainsi 4,6 g de mélange de cyano-6 et de cyano-7 (thiazolyl-5)-5 1H,3H-pyrrolo [1,2-c] thiazole, sous forme de cristaux marron fondant à 155°C, dans un rapport 20-80 (d'après le spectre de RMN).

EXEMPLE 24

Une solution de 5,5 g de méthyl-7 (pyridyl-3)-6 dihydro-1,2 4H-pyrrolo [1,2-c] thiazine-1,3 carbonitrile-8 dans 70 cm3 d'une solution aqueuse d'acide chlorhydrique 12N est chauffée sous agitation à une température comprise entre 80 et 86°C pendant 1 heure et 30 minutes puis à une température voisine de 96°C pendant 7 heures et 30 minutes. La solution obtenue est agitée à une température voisine de 20°C pendant 16 heures puis est additionnée de 20 cm3 d'une solution aqueuse d'acide chlorhydrique 12N et chauffée à une température voisine de 98°C pendant 6 heures et 30 minutes. La solution obtenue est additionnée de 100 cm3 d'eau distillée et amenée à un pH voisin de 9 par addition de carbonate de potassium. La suspension obtenue est extraite 3 fois par 450 cm3 au total d'un mélange de chlorure de méthylène et de méthanol (9-1 en volumes). Les extraits organiques sont réunis, lavés 2 fois par 100 cm3 au total d'eau distillée, séchés sur du sulfate de magnésium anhydre, additionnés de 0,5 g de noir décolorant, filtrés et concentrés sous pression réduite (20 mm de mercure; 2,7 kPa) à une température voisine de 50°C. On obtient ainsi 4,8 g de produit brut que l'on

chromatographie sur une colonne de 6 cm de diamètre contenant 480 g de silice (0,04 - 0,063 mm). On élue par un mélange d'acétate d'éthyle et de méthanol (95-5 en volumes) sous une pression de 0,5 bar (51 kPa) et en recueillant des fractions de 200 cm3. Les 9 premières fractions sont éliminées, les 5 fractions suivantes sont réunies et concentrées à sec sous pression réduite (20 mm de mercure; 2,7 kPa) à une température voisine de 50°C. On obtient ainsi 1,8 g de produit que l'on réunit à 0,3 g de produit préparé de la même façon lors d'une opération antérieure et dissout dans 60 cm3 d'éthanol bouillant. La solution obtenue est additionnée de 0,5 g de noir décolorant et filtrée à chaud. Le filtrat est refroidi à une température voisine de 4°C pendant 16 heures. Les cristaux apparus sont séparés par filtration, lavés 2 fois par 4 cm3 au total d'éthanol refroidi à une température voisine de 4°C et 3 fois par 12 cm3 au total d'éther diéthylique puis séchés sous pression réduite (20 mm de mercure; 2,7 kPa) à une température voisine de 20°C en présence de potasse en pastilles. On obtient ainsi 1,3 g de méthyl-7 (pyridyl-3)-6 dihydro-1,2 4H-pyrrolo [1,2-c] thiazine-1,3 carboxamide-8 sous forme de cristaux blancs fondant à 220°C.

Le méthyl-7 (pyridyl-3)-6 dihydro-1,2 4H-pyrrolo [1,2-c] thiazine-1,3 carbonitrile-8 peut être préparé de la façon suivante :

A une solution de 9 g d'acide N-nicotinoyl tétrahydro-3,4,5,6 2H-thiazine-1,3 carboxylique-4 dans 100 cm3 d'anhydride acétique, on ajoute en 8 minutes, à une température voisine de 80°C, 36 g de chloro-2 crotononitrile. La solution obtenue est chauffée à une température comprise entre 85 et 90°C pendant 3 heures puis le solvant est évaporé sous pression réduite (20 mm de mercure; 2,7 kPa) à une température voisine de 80°C. L'huile résiduelle est dissoute dans 100 cm3 d'eau distillée et la solution obtenue est amenée à un pH voisin de 10 par addition de carbonate de potassium solide puis de 10 cm3 d'une solution aqueuse de soude 4N. Cette solution basique est extraite 5 fois par 500 cm3 au total d'acétate d'éthyle. Les extraits organiques sont réunis, lavés 5 fois par 400 cm3 au total d'eau distillée, séchés sur du sulfate de magnésium anhydre, additionnés de 0,5 g de noir décolorant, filtrés et

concentrés à sec sous pression réduite (20 mm de mercure; 2,7 kPa) à une température voisine de 50°C. On obtient ainsi 6,9 g de produit brut fondant à 130°C, qui, réuni à 4,3 g de produit obtenu de la même façon dans une autre opération est dissous dans 60 cm3 d'isopropanol bouillant. La solution obtenue est additionnée de 0,5 g de noir décolorant et filtrée à chaud. Le filtrat est refroidi à une température voisine de 4°C pendant 16 heures. Les cristaux apparus sont séparés par filtration, lavés 3 fois par 15 cm3 au total d'isopropanol refroidi à une température voisine de 4°C et 3 fois par 30 cm3 au total d'oxyde d'isopropyle puis séchés sous pression réduite (20 mm de mercure; 2,7 kPa) à une température voisine de 20°C en présence de potasse en pastilles. On obtient ainsi 4,8 g de méthyl-7 (pyridyl-3)-6 dihydro-1,2 4H-pyrrrolo [1,2-c] thiazine-1,3 carbonitrile-8 sous forme de cristaux de couleur ocre fondant à 166°C.

L'acide N-nicotinoyl tétrahydro-3,4,5,6 2H-thiazine-1,3 carboxylique-4 est préparé comme décrit dans la demande de brevet européen publiée sous le numéro 0 118 321.

Le chloro-2 crotononitrile peut être préparé selon J.C. POMMELET, C. NYNS, F. LAHOUSSE, R. MERENYI et H.G. VIEHE, Angew. Chem. Int. Ed. 20, 585 (1981).

EXEMPLE 25

A une solution de 16,2 g d'acide N-nicotinoyl tétrahydro-3,4,5,6 2H-thiazine-1,3 carboxylique-4 dans 160 cm3 d'anhydride acétique, on ajoute en 2 minutes, à une température voisine de 85°C, une solution de 14 g de N-phényl propiolamide dans 60 cm3 d'anhydride acétique. La solution obtenue est chauffée à une température voisine de 85°C pendant 1 heure puis le solvant est évaporé sous pression réduite (20 mm de mercure; 2,7 kPa) à une température voisine de 70°C. L'huile résiduelle est mise en suspension dans 310 cm3 d'une solution aqueuse de soude 0,4N et le mélange obtenu est extrait 3 fois par 750 cm3 au total d'acétate d'éthyle. Les extraits organiques sont réunis, lavés 2 fois par 400 cm3 au total d'eau

distillée, séchés sur du sulfate de magnésium anhydre, additionnés de 0,5 g de noir décolorant, filtrés et concentrés à sec sous pression réduite (20 mm de mercure; 2,7 kPa) à une température voisine de 50°C. On obtient ainsi 23,5 g de produit que l'on chromatographie sur une colonne de 6 cm de diamètre contenant 480 g de silice (0,04 - 0,063 mm). On élue par un mélange d'acétate d'éthyle et de cyclohexane (80-20 en volumes) sous une pression de 0,5 bar et en recueillant des fractions de 200 cm3. Les 8 premières fractions sont éliminées. Les 6 fractions suivantes sont réunies, concentrées à sec sous pression réduite (20 mm de mercure; 2,7 kPa) à une température voisine de 50°C. On obtient 5,8 g de produit. Les 7 fractions suivantes sont réunies, concentrées à sec sous pression réduite (20 mm de mercure; 2,7 kPa) à une température voisine de 50°C. On obtient ainsi 4,2 g de produit que l'on chromatographie à nouveau sur une colonne de 6 cm de diamètre contenant 480 g de silice (0,04 - 0,063 mm). On élue par un mélange d'acétate d'éthyle et de cyclohexane (80-20 en volumes) sous une pression de 0,5 bar et en recueillant des fractions de 200 cm3. Les 8 premières fractions sont éliminées, les 4 fractions suivantes sont réunies, concentrées à sec sous pression réduite (20 mm de mercure; 2,7 kPa) à une température voisine de 50°C. On obtient ainsi 1,8 g de produit, que l'on ajoute aux 5,8 g précédemment obtenus et à 0,5 g de produit préparé de la même façon dans une autre opération; ce lot unique est repris par 100 cm3 d'isopropanol bouillant. La solution obtenue est additionnée de 0,5 g de noir décolorant et filtrée à chaud. Le filtrat est refroidi à une température voisine de 4°C pendant 30 minutes. Les cristaux apparus sont séparés par filtration, lavés 2 fois par 20 cm3 au total d'isopropanol refroidi à une température voisine de 4°C et 3 fois par 60 cm3 au total d'oxyde d'isopropyle puis séchés sous pression réduite (20 mm de mercure; 2,7 kPa) à une température voisine de 20°C en présence de potasse en pastilles. On obtient ainsi 4,6 g de N-phényl (pyridyl-3)-6 dihydro-1,2 4H-pyrrolo [1,2-c] thiazine-1,3 carboxamide-8 sous forme de cristaux orangés fondant à 160°C.

L'acide N-nicotinoyl tétrahydro-3,4,5,6 2H-thiazine-1,3 carboxylique-4 est préparé comme décrit dans la demande de brevet européen publié sous le numéro 0118 321.

Le N-phényl propiolamide peut être préparé de la façon suivante :

A une solution de 28 g d'acide propiolique dans 170 cm3 de tétrahydrofuranne anhydre, on ajoute en 20 minutes à une température voisine de 4°C, une solution de 82,4 g de dicyclohexylcarbodiimide dans 170 cm3 de tétrahydrofuranne anhydre et agite la suspension obtenue pendant 30 minutes à une température voisine de 4°C. On ajoute ensuite en 25 minutes une solution de 37,9 g d'aniline dans 170 cm3 de tétrahydrofuranne anhydre en maintenant la même température et agite la suspension à une température voisine de 4°C pendant 1 heure puis à une température voisine de 20°C pendant 16 heures. Les cristaux sont filtrés, lavés 3 fois par 90 cm3 au total de tétrahydrofuranne et éliminés. Le filtrat est concentré à sec sous pression réduite (20 mm de mercure; 2,7 kPa) à une température voisine de 40°C. L'huile résiduelle est dissoute dans 350 cm3 de chlorure de méthylène, la solution obtenue est lavée 5 fois par 400 cm3 au total d'une solution aqueuse d'acide chlorhydrique N, 3 fois par 300 cm3 au total d'eau distillée, 5 fois par 400 cm3 au total d'une solution aqueuse de soude N et 5 fois par 500 cm3 au total d'eau distillée, séchée sur du sulfate de magnésium anhydre, filtrée et concentrée à sec sous pression réduite (20 mm de mercure; 2,7 kPa) à une température voisine de 40°C. On obtient ainsi 63 g de produit que l'on chromatographie sur une colonne de 5,3 cm de diamètre contenant 600 g de silice (0,063 - 0,2 mm) en éluant par du chlorure de méthylène et en recueillant des fractions de 1 litre. Les 2 premières fractions sont éliminées. Les 8 fractions suivantes sont réunies, concentrées à sec sous pression réduite (20 mm de mercure; 2,7 kPa) à une température voisine de 40°C. On obtient ainsi 24,5 g de produit que l'on dissout dans 75 cm3 de tétrachlorure de carbone bouillant. La solution obtenue est refroidie à une température voisine de 4°C pendant 1 heure. Les cristaux apparus sont séparés par filtration, lavés 3 fois par 25 cm3 au total de tétrachlorure de carbone refroidi à une température voisine de 4°C

et séchés sous pression réduite (20 mm de mercure; 2,7 kPa) à une température voisine de 20°C en présence de potasse en pastilles. On obtient ainsi 20 g de N-phénylpropiolamide sous forme de cristaux crème fondant à 86°C.

EXEMPLE 26

A une suspension de 7,5 g de chlorhydrate de chloroformyl-7 (pyridyl-3)-5 1H,3H-pyrrolo [1,2-c] thiazole dans 125 cm3 de chlorure de méthylène, on ajoute en 20 minutes à une température comprise entre 21°C et 29°C une solution de 4 g de trifluorométhyl-3 aniline et de 5 g de triéthylamine dans 50 cm3 de chlorure de méthylène. La solution obtenue est agitée à une température voisine de 20°C pendant 16 heures puis est additionnée de 200 cm3 de chlorure de méthylène et de 150 cm3 d'eau distillée. La phase organique est décantée, lavée 2 fois par 300 cm3 au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 5,6 g de produit fondant à 190°C. On dissout ce produit dans 160 cm3 d'éthanol bouillant; la solution obtenue est additionnée de 0,5 g de noir décolorant et filtrée à chaud. Le filtrat est refroidi à une température voisine de 4°C pendant 2 heures. Les cristaux apparus sont séparés par filtration, lavés 2 fois par 50 cm3 au total d'éthanol et 3 fois par 75 cm3 au total d'éther diéthylique puis séchés sous pression réduite (20 mm de mercure; 2,7 kPa) à une température voisine de 20°C en présence de potasse en pastilles. On obtient ainsi 2,9 g de N-(trifluorométhyl-3 phényl) (pyridyl-3)-5 1H,3H-pyrrolo [1,2-c] thiazolecarboxamide-7 sous forme de cristaux jaune pâle fondant à 198°C.

Le chlorhydrate de chloroformyl-7 (pyridyl-3)-5 1H,3H-pyrrolo [1,2-c] thiazole est préparé comme à l'exemple 1.

EXEMPLE 27

Une solution de 4,7 g d'hydroxy-2 (pyridyl-3)-5 dihydro-2,3 1H-pyrrolizinecarbonitrile-7 dans 110 cm3 d'une solution aqueuse d'acide chlorhydrique 12N est chauffée à une température voisine de 80°C pendant 2 heures puis est concentrée à sec sous pression réduite (20 mm de mercure; 2,7 kPa) à une température voisine de 60°C. On obtient ainsi 8 g de produit que l'on dissout dans 20 cm3 d'eau distillée. La solution résultante est amenée à un pH voisin de 8 par addition de 100 cm3 d'une solution aqueuse saturée d'hydrogénocarbonate de sodium 1,19N puis est concentrée à sec sous pression réduite (20 mm de mercure; 2,7 kPa) à une température voisine de 60°C. Le solide obtenu est repris une première fois avec 500 cm3 d'un mélange de chlorure de méthylène et de méthanol (80-20 en volumes). On obtient ainsi une solution et un insoluble. La solution est décantée avec précaution et versée sur une colonne de 13 cm de diamètre contenant 400 g de silice (0,063 - 0,2 mm). L'insoluble est repris à nouveau par 500 cm3 d'un mélange de chlorure de méthylène et de méthanol (80-20 en volumes) et traité comme précédemment. On recommence ainsi l'opération 9 fois de suite. Les solutions organiques que l'on recueille en bas de la colonne sont réunies et concentrées à sec sous pression réduite (20 mm de mercure; 2,7 kPa) à une température voisine de 60°C. On obtient ainsi 4,7 g de produit que l'on réunit à 0,5 g de produit préparé de la même façon dans une autre opération et dissout dans 140 cm3 d'éthanol bouillant. La solution obtenue est additionnée de 0,5 g de noir décolorant et filtrée à chaud. Le filtrat est refroidi à une température voisine de 4°C pendant 16 heures. Les cristaux apparus sont séparés par filtration, lavés 2 fois par 50 cm3 au total d'éthanol et 3 fois par 75 cm3 au total d'éther diéthylique puis séchés sous pression réduite (20 mm de mercure; 2,7 kPa) à une température voisine de 20°C en présence de potasse en pastilles. On obtient ainsi 3,3 g d'hydroxy-2 (pyridyl-3)-5 dihydro-2,3 1H-pyrrolizinecarboxamide-7 sous forme de cristaux crème fondant à 206°C.

L'hydroxy-2 (pyridyl-3)-5 dihydro-2,3 1H-pyrrolizinecarbonitrile-7 peut être préparé de la façon suivante :

Une suspension de 34 g de N-nicotinoyl hydroxy-4 proline dans un mélange de 115 cm3 de chloro-2 acrylonitrile et de 150 cm3 d'anhydride acétique est chauffée à une température voisine de 90°C pendant 3 heures et 40 minutes. La solution obtenue est concentrée à sec sous pression réduite (20 mm de mercure; 2,7 kPa) à une température voisine de 70°C. Le résidu obtenu est mis en suspension dans 500 cm3 d'une solution aqueuse de soude 5N et la suspension résultante est extraite 4 fois par 1250 cm3 au total d'acétate d'éthyle. Les extraits organiques sont réunis, lavés 4 fois par 1000 cm3 au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 60°C. On obtient ainsi 13 g de produit brut que l'on dissout dans 130 cm3 d'isopropanol bouillant. La solution obtenue est filtrée à chaud et le filtrat est refroidi à une température voisine de 20°C pendant 2 heures. Les cristaux apparus sont séparés par filtration, lavés 2 fois par 50 cm3 au total d'isopropanol et 3 fois par 150 cm3 au total d'éther diéthylique puis séchés sous pression réduite (20 mm de mercure; 2,7 kPa) à une température voisine de 20°C en présence de potasse en pastilles. On obtient ainsi 5,6 g d'hydroxy-2 (pyridyl-3)-5 dihydro-2,3 1H-pyrrolizinecarbonitrile-7 sous forme de cristaux beiges fondant à 190°C.

La N-nicotinoyl hydroxy-4 proline peut être préparée de la façon suivante :

A une suspension de 212,8 g de bis 0,N-nicotinoyl hydroxy-4 prolinate d'éthyle dans 2130 cm3 d'éthanol, on ajoute en 25 minutes à une température comprise entre 14°C et 26°C 346 cm3 d'une solution aqueuse de soude 5N. La solution obtenue est agitée à une température voisine de 20°C pendant 16 heures puis est amenée à un pH voisin de 3 par addition de 160 cm3 d'une solution aqueuse d'acide chlorhydrique 12N. Les cristaux apparus sont éliminés par filtration et le filtrat est concentré à sec sous pression réduite

(20 mm de mercure; 2,7 kPa) à une température voisine de 70°C. Le résidu obtenu est mis en suspension dans 1250 cm3 d'éthanol bouillant. Après refroidissement à une température voisine de 20°C, les cristaux sont éliminés par filtration et le filtrat est concentré à la moitié de son volume sous pression réduite (20 mm de mercure; 2,7 kPa) à une température voisine de 50°C. La solution obtenue est refroidie à une température voisine de 4°C pendant 16 heures. Les cristaux apparus sont séparés par filtration, lavés 3 fois par 300 cm3 au total d'éthanol et 3 fois par 450 cm3 au total d'éther diéthylique puis séchés sous pression réduite (20 mm de mercure; 2,7 kPa) à une température voisine de 20°C en présence de potasse en pastilles. On obtient ainsi 112,6 g de N-nicotinoyl hydroxy-4 proline sous forme de cristaux rose pâle fondant à 172°C.

La bis O,N-nicotinoyl hydroxy-4 proline peut être préparée de la façon suivante :

A une suspension de 209,6 g de chlorhydrate d'hydroxy-4 prolinate d'éthyle dans 2800 cm3 de chlorure de méthylène, on ajoute en 10 minutes, 655 g de triéthylamine en maintenant la température au voisinage de 24°C. Durant cette période, on observe un passage par une phase homogène limpide au bout de 5 minutes, immédiatement suivi d'une précipitation. A la suspension obtenue, on ajoute en 15 minutes, à une température comprise entre 23°C et 43°C, 480,6 g de chlorhydrate de chlorure de nicotinoyle. La suspension obtenue est chauffée à une température voisine de 43°C pendant 3 heures puis est agitée à une température voisine de 20°C pendant 16 heures. On ajoute alors à la suspension 1000 cm3 de chlorure de méthylène et 2000 cm3 d'eau. La phase organique est séparée par décantation, lavée 3 fois par 3000 cm3 au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 458,7 g de produit brut fondant à 80°C. Ce produit est repris par 2000 cm3 d'eau distillée à l'ébullition. L'huile insoluble apparue est séparée par décantation à chaud et la phase aqueuse est filtrée à chaud. Le filtrat est refroidi à une température voisine de 4°C pendant 16 heures. Les cristaux apparus sont séparés par

filtration, lavés 3 fois par 750 cm3 au total d'eau distillée et séchés à l'air. On obtient ainsi 212,8 g de bis O,N-nicotinoyl hydroxy-4 prolinate d'éthyle sous forme de cristaux jaune pâle fondant à 110°C.

Le chlorhydrate d'hydroxy-4 prolinate d'éthyle peut être préparé selon J. KAPFHAMMER et A.MATTHES, Z. Physiol. Chem., 223, 48 (1934).

EXEMPLE 28

A une suspension de 7,5 g de chlorhydrate de chloroformyl-7 (pyridyl-3)-5 1H,3H-pyrrolo [1,2-c] thiazole dans 125 cm3 de chlorure de méthylène, on ajoute en 15 minutes à une température comprise entre 21 et 28°C une solution de 3,1 g de méthoxy-3 aniline et de 5,1 g de triéthylamine dans 50 cm3 de chlorure de méthylène. La solution obtenue est agitée à une température voisine de 20°C pendant 16 heures. Un produit précipite. Les cristaux apparus sont séparés par filtration, lavés 3 fois par 300 cm3 au total de chlorure de méthylène et 3 fois par 300 cm3 au total d'eau distillée puis séchés sous pression réduite (20 mm de mercure; 2,7 kPa) à une température voisine de 20°C en présence de potasse en pastilles. On obtient ainsi 5,1 g de produit brut fondant à 220°C. On dissout ce produit dans 160 cm3 de butanol-1 bouillant. La solution trouble obtenue est additionnée de 0,5 g de noir décolorant et filtrée à chaud. Le filtrat est refroidi à une température voisine de 4°C pendant 16 heures. Les cristaux apparus sont séparés par filtration, lavés 2 fois par 30 cm3 au total de butanol-1, 3 fois par 75 cm3 au total d'éthanol et 3 fois par 75 cm3 au total d'éther diéthylique puis séchés sous pression réduite (20 mm de mercure; 2,7 kPa) à une température voisine de 20°C en présence de potasse en pastilles. On obtient ainsi 3,9 g de N-(méthoxy-3 phényl) (pyridyl-3)-5 1H,3H-pyrrolo [1,2-c] thiazolecarboxamide-7 sous forme de cristaux jaune pâle fondant à 224°C.

Le chlorhydrate de chloroformyl-7 (pyridyl-3)-5 1H,3H-pyrrolo [1,2-c] thiazole est préparé comme à l'exemple 1.

EXEMPLE 29

Une suspension de 6 g d'un mélange (dans la proportion 10-90) de chlorhydrate de cyano-6 et de cyano-7 (pyridyl-3)-5 dihydro-2,3 pyrrolo [2,1-b] thiazole et de 7,5 g de potasse en poudre dans 140 cm3 d'alcool tert-butylique est chauffée à une température voisine de 82°C pendant 1 heure et 10 minutes. Le mélange réactionnel est concentré à sec sous pression réduite (20 mm de mercure; 2,7 kPa) à une température voisine de 50°C et le résidu obtenu est mis en suspension dans 100 cm3 d'eau distillée. Les cristaux apparus sont séparés par filtration, lavés 5 fois par 200 cm3 au total d'eau distillée et 3 fois par 30 cm3 au total d'éthanol puis séchés sous pression réduite (20 mm de mercure; 2,7 kPa) à une température voisine de 20°C. On obtient ainsi 3 g de produit brut fondant à 251°C. On ajoute à ce produit 1,4 g de produit préparé de la même façon lors d'une autre opération et dissout le lot unique obtenu dans 37 cm3 de diméthylformamide chauffé à une température voisine de 125°C. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 3 fois par 3 cm3 au total de diméthylformamide refroidi à une température voisine de 4°C, 5 fois par 15 cm3 au total d'éthanol refroidi à une température voisine de 4°C et 5 fois par 25 cm3 au total d'éther diéthylique puis séchés sous pression réduite (20 mm de mercure; 2,7 kPa) à une température voisine de 20°C en présence de potasse en pastilles. On obtient ainsi 3 g de (pyridyl-3)-5 dihydro-2,3 pyrrolo [2,1-b] thiazolecarboxamide-7 sous forme de cristaux crème fondant à 253°C.

Le mélange (dans la proportion 10-90) de chlorhydrate de cyano-6 et de cyano-7 (pyridyl-3)-5 dihydro-2,3 pyrrolo [2,1-b] thiazole peut être préparé de la façon suivante :

Une suspension de 9,5 g d'acide N-nicotinoyl thiazolidinecarboxylique-2 et de 35 g de chloro-2 acrylonitrile dans 100 cm3 d'anhydride acétique est chauffée à une température comprise entre 88°C et 108°C pendant 2 heures. Durant cette période, on observe un passage par une phase homogène limpide au bout de 30 minutes suivi

d'une précipitation 25 minutes plus tard. Après refroidissement à une température voisine de 20°C pendant 16 heures, les cristaux apparus sont séparés par filtration, lavés 2 fois par 10 cm3 au total d'anhydride acétique et 2 fois par 20 cm3 au total d'éther diéthylique puis séchés sous pression réduite (20 mm de mercure; 2,7 kPa) à une température voisine de 20°C en présence de potasse en pastilles. On obtient ainsi 8,6 g de mélange de chlorhydrate de cyano-6 et de cyano-7 (pyridyl-3)-5 dihydro-2,3 pyrrolo [2,1-b] thiazole sous forme de cristaux marron fondant à 160°C, dans un rapport 10-90 (d'après le spectre de R.M.N.).

L'acide N-nicotinoyl thiazolidinecarboxylique-2 peut être préparé de la façon suivante :

A une suspension de 77 g d'acide thiazolidinecarboxylique-2 dans 700 cm3 de chloroforme, on ajoute en 5 minutes, à une température comprise entre 22 et 30°C, 133,6 g de triéthylamine. A la solution obtenue, on ajoute en une heure, à une température comprise entre 23 et 40°C, 117,5 g de chlorhydrate de chlorure de nicotinoyle. La solution obtenue est chauffée à une température voisine de 64°C pendant 2 heures. Après refroidissement à une température voisine de 20°C pendant 16 heures puis à une température voisine de 4°C pendant 1 heure, les cristaux apparus sont séparés par filtration, lavés 3 fois par 300 cm3 au total de chloroforme refroidi à une température voisine de 4°C et éliminés. Le filtrat est concentré à sec sous pression réduite (20 mm de mercure; 2,7 kPa) à une température voisine de 65°C. Le résidu est mis en suspension dans 1000 cm3 d'acétone et les cristaux apparus sont séparés par filtration, lavés 3 fois par 750 cm3 au total d'acétone et éliminés. Le filtrat est concentré à sec sous pression réduite (20 mm de mercure; 2,7 kPa) à une température voisine de 60°C. On obtient 150 g de produit. Ce produit est dissous dans 500 cm3 d'eau distillée et la solution est amenée à un pH voisin de 9 par addition d'une solution aqueuse de soude 10N; la solution est versée sur une colonne de 6 cm de diamètre contenant 1 kg de résine DOWEX 50 W X 2 forme $H^+$ (50-100 mesh). On élue par de l'eau distillée en recueillant des fractions de 1000 cm3. Les deux premières fractions sont éliminées. Les quatre fractions suivantes sont réunies et

concentrées jusqu'à un volume de 150 cm3 sous pression réduite (20 mm de mercure; 2,7 kPa) à une température voisine de 60°C. La solution obtenue est abandonnée à une température voisine de 20°C pendant 16 heures. Un produit précipite. Les cristaux apparus sont séparés par filtration, lavés 2 fois par 10 cm3 au total d'eau distillée, 2 fois par 10 cm3 au total d'éthanol puis séchés sous pression réduite (20 mm de mercure; 2,7 kPa) à une température voisine de 20°C en présence de potasse en pastilles. On obtient ainsi 9,5 g d'acide N-nicotinoyl thiazolidinecarboxylique-2 sous forme de cristaux blancs fondant à 180°C.

L'acide thiazolidinecarboxylique-2 peut être préparé selon R.L.JOHNSON, E.E. SMISSMAN et N.P. PLOTNIKOFF, J. Med., Chem. 21, 165 (1978).

EXEMPLE 30

A une suspension de 12 g de chlorhydrate de chloroformyl-7 (pyridyl-3)-5 1H,3H-pyrrolo [1,2-c] thiazole dans 200 cm3 de chlorure de méthylène, on ajoute en 15 minutes, à une température comprise entre 20 et 31°C, une solution de 11,9 g de cyclohexylamine dans 50 cm3 de chlorure de méthylène. La solution obtenue est agitée à une température voisine de 20°C pendant 16 heures puis est additionnée de 250 cm3 de chlorure de méthylène et de 250 cm3 d'eau distillée. La phase organique est séparée par décantation, lavée 3 fois par 750 cm3 au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 13,1 g de produit fondant à 180°C que l'on dissout dans 200 cm3 d'éthanol bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 50 cm3 au total d'éthanol refroidi à une température voisine de 4°C et 3 fois par 75 cm3 au

total d'éther diéthylique puis séchés sous pression réduite (20 mm de mercure; 2,7 kPa) à une température voisine de 20°C en présence de potasse en pastilles. On obtient ainsi 4,5 g de N-cyclohexyl (pyridyl-3)-5 1H,3H-pyrrolo [1,2-c] thiazolecarboxamide-7 sous forme de cristaux beiges fondant à 188°C.

Le chlorhydrate de chloroformyl-7 (pyridyl-3)-5 1H,3H-pyrrolo [1,2-c] thiazole est préparé comme à l'exemple 1.

La présente invention concerne également les médicaments constitués par un produit de formule générale (I), sous forme libre ou sous forme de sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale, rectale ou topique.

Comme compositions solides pour administration orale peuvent être utilisés des comprimés, pilules, poudres (notamment dans des capsules de gélatine ou des cachets) ou granulés. Dans ces compositions, le produit actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, les glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parantérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'empmoi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les produits selon l'invention sont particulièrement utiles dans le traitement prophylactique et thérapeutique des affections thrombotiques. Les doses dépendent de l'effet recherché et de la durée du traitement; elles sont généralement comprises entre 100 et 1000 mg par jour par voie orale pour un adulte en une ou plusieurs prises et entre 10 et 100 mg par voie parentérale pour un adulte en une ou plusieurs injections.

D'une façon générale, le médecin déterminera la posologie qu'il estime la plus appropriée en fonction de l'âge, du poids et de tous les autres facteurs propres au sujet à traiter.

Les exemples suivants, donnés à titre non limitatif, illustrent des compositions selon l'invention.

Exemple A

On prépare, selon la technique habituelle, des comprimés dosés à 200 mg de produit actif ayant la composition suivante :

- N-(adamantyl-1) (pyridyl-3)-5 1H,3H-pyrrolo [1,2-c] thiazolecarboxamide-7............................ 200 mg
- amidon.......................................... 60 mg
- lactose ........................................ 50 mg
- stéarate de magnésium........................... 2 mg

Exemple B

On prépare, selon la technique habituelle, des comprimés dosés à 200 mg de produit actif ayant la composition suivante :

- N(chloro-3 phényl) (pyridyl-3)-5 1H,3H-pyrrolo [1,2-c] thiazolecarboxamide-7..................... 200 mg
- amidon.......................................... 60 mg
- lactose.......................................... 50 mg
- stéarate de magnésium........................... 2 mg

Exemple C

On prépare une solution injectable contenant 20 mg de produit actif ayant la composition suivante :

- N-(pyridyl-3) (pyridyl-3)-5 1H,3H-pyrrolo [1,2-c] thiazolecarboxamide-7............................ 20 mg
- acide méthanesulfonique 0,1N.................... 0,62 cm3
- soluté injectable q.s.p......................... 2 cm3

# R E V E N D I C A T I O N S

1 - Nouveau dérivé orthocondensé du pyrrole, caractérisé en ce qu'il répond à la formule générale :

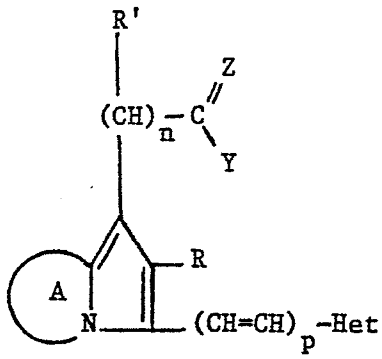

(I)

dans laquelle R' représente un atome d'hydrogène ou un radical alcoyle ou phényle éventuellement substitué par un atome d'halogène ou un radical alcoyle, alcoyloxy ou alcoylthio, Z représente un atome d'oxygène ou de soufre, p représente le nombre entier zéro ou 1 et

A - soit le symbole A représente un hétérocycle tel qu'avec le noyau pyrrole auquel il est condensé, il forme un cycle 1H,3H-pyrrolo [1,2-c] thiazole, dihydro-2,3 1H-pyrrolizine éventuellement substitué par un radical hydroxy, tétrahydro-5,6,7,8 indolizine, dihydro-1,2 4H-pyrrolo [1,2-c] thiazine-1,3, dihydro-2,3 pyrrolo [2,1-b] thiazole, 1H,3H-pyrrolo [1,2-c] oxazole, dihydro-1,2 4H-pyrrolo [1,2-c] oxazine-1,3 ou dihydro-2,3 pyrrolo [2,1-b] oxazole, n représente le nombre entier 0 ou 1, Het représente un radical pyridyle-3 ou thiazolyle-5 et

1) ou bien R représente un atome d'hydrogène ou d'halogène ou un radical alcoyle ou phényle éventuellement substitué par un atome d'halogène ou un radical alcoyle , alcoyloxy ou alcoylthio et Y représente un radical alcoyle ou phényle éventuellement substitué par un atome d'halogène ou un radical alcoyle, alcoyloxy ou alcoylthio, ou encore Y représente un radical de formule générale :

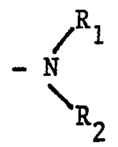 (II)

dans laquelle

- soit $R_1$ représente un atome d'hydrogène et $R_2$ représente un radical alcoyle non substitué, cycloalcoyle contenant 3 à 6 atomes de carbone, alcényle contenant 2 à 4 atomes de carbone, alcynyle contenant 3 ou 4 atomes de carbone, benzyle ou phényle éventuellement substitués par un atome d'halogène ou un radical alcoyle, alcoyloxy, alcoylthio, trifluorométhyle ou nitro, ou bien $R_2$ représente un radical adamantyle, pyridyle ou pyridylméthyle,

- soit $R_1$ et $R_2$ représentent tous deux un radical alcoyle non substitué,

- soit $R_1$ et $R_2$ forment avec l'atome d'azote auquel ils sont liés un radical phényl-4 pipérazinyle-1 dont la partie phényle peut être éventuellement substituée par un atome d'halogène ou un radical alcoyle, alcoyloxy, alcoylthio, trifluorométhyle ou nitro,

2) ou bien R représente un atome d'halogène ou un radical alcoyle ou phényle éventuellement substitué par un atome d'halogène ou un radical alcoyle, alcoyloxy ou alcoylthio, et Y représente un radical amino,

B - soit le symbole A représente un hétérocycle tel qu'avec le noyau pyrrole auquel il est condensé, il forme un cycle dihydro-2,3 1H-pyrrolizine substitué par un radical hydroxy, dihydro-2,3 pyrrolo [2,1-b] thiazole ou dihydro-2,3 pyrrolo [2,1-b] oxazole, R représente un atome d'hydrogène, Y représente un radical amino, n représente le nombre zéro ou 1 et Het représente un radical thiazolyle-5 ou pyridyle-3,

C - soit le symbole A représente un hétérocycle tel qu'avec le noyau pyrrole auquel il est condensé, il forme un cycle 1H,3H-pyrrolo [1,2-c] thiazole, dihydro-2,3 1H-pyrrolizine, tétrahydro-5,6,7,8 indolizine, dihydro-1,2 4H-pyrrolo [1,2-c] thiazine-1,3, 1H,3H-pyrrolo [1,2-c] oxazole ou dihydro-1,2 4H-pyrrolo [1,2-c] oxazine-1,3, R représente un atome d'hydrogène, Y représente un radical amino et

- soit Het représente le radical thiazolyle-5 et n est égal à zéro ou 1,
- soit Het représente le radical pyridyle-3 et n est égal à 1, étant entendu que, sauf mention spéciale, les radicaux alcoyle et portions alcoyle contiennent 1 à 4 atomes de carbone et sont en chaîne droite ou ramifiée,

ainsi que ses sels d'addition avec les acides.

2 - Procédé de préparation d'un produit selon la revendication 1 dans la formule duquel Z représente un atome d'oxygène, Y représente un radical alcoyle ou phényle éventuellement substitué par un atome d'halogène ou un radical alcoyle, alcoyloxy ou alcoylthio et les autres symboles sont définis comme à la revendication 1, caractérisé en ce que l'on fait agir un dérivé organomagnésien de formule générale :

$$Y'-MgX_1 \qquad (III)$$

dans laquelle Y' représente un radical alcoyle ou phényle éventuellement substitué par un atome d'halogène ou un radical alcoyle, alcoyloxy ou alcoylthio et $X_1$ représente un atome d'halogène, sur un nitrile de formule générale :

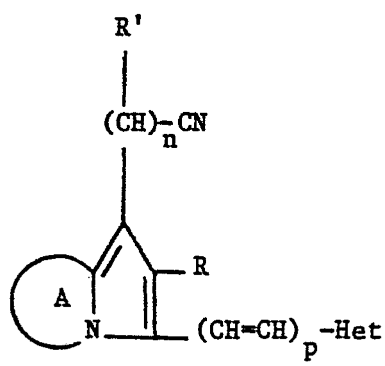

(IV)

dans laquelle les symboles sont définis comme à la revendication 1, puis isole le produit obtenu et le transforme éventuellement en un sel d'addition avec un acide.

3 - Procédé de préparation d'un produit selon la revendication 1 dans la formule duquel les symboles sont définis comme à la revendication 1, à l'exception pour Z de représenter un atome de soufre et pour Y de représenter un radical alcoyle ou phényle éventuellement substitué par un atome d'halogène ou un radical alcoyle, alcoyloxy ou alcoylthio, caractérisé en ce que l'on fait réagir l'ammoniac ou une amine de formule générale :

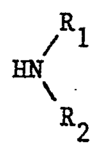

(XIV)

dans laquelle $R_1$ et $R_2$ sont définis comme à la revendication 1 sur un acide de formule générale :

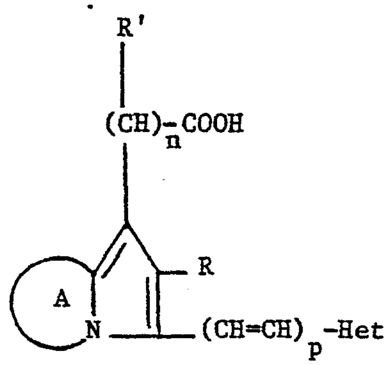

(XV)

dans laquelle les différents symboles sont définis comme à la revendication 1, puis isole le produit obtenu et le transforme éventuellement en un sel d'addition avec un acide.

4 - Procédé de préparation d'un produit selon la revendication 1 dans la formule duquel Z représente un atome d'oxygène, Y représente un radical méthyle et les autres symboles sont définis comme à la revendication 1, caractérisé en ce que l'on fait agir l'éthoxymagnésien du malonate d'éthyle sur un halogènure de formule générale :

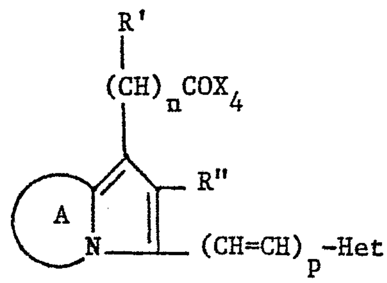

(XVI)

dans laquelle $X_4$ représente un atome d'halogène, R" a la définition donnée à la revendication 1 pour R et les autres symboles sont définis comme à la revendication 1, puis isole le produit et le transforme éventuellement en un sel d'addition avec un acide.

5 - Procédé de préparation d'un produit selon la revendication 1 dans la formule duquel Z représente un atome de soufre et les autres symboles sont définis comme à la revendication 1, caractérisé en ce que l'on procède à la thionation d'un produit selon la revendication 1 dans la formule duquel Z représente un atome d'oxygène et les autres symboles sont définis comme à la revendication 1, c'est-à-dire un produit de formule générale :

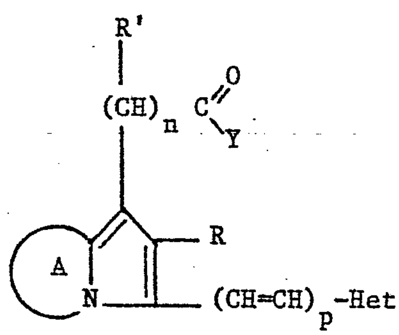

(XVII)

par toute méthode connue pour transformer un amide ou une cétone respectivement en thioamide ou thiocétone, puis isole le produit et le transforme éventuellement en un sel d'addition avec un acide.

6 - Procédé de préparation d'un produit selon la revendication 1 dans la formule duquel Z représente un atome de soufre, Y représente un radical amino et R représente un radical alcoyle ou phényle éventuellement substitué par un atome d'halogène ou un radical alcoyle alcoyloxy ou alcoylthio, caractérisé en ce que l'on transforme en thioamide un nitrile défini comme à la revendication 2 par toute méthode connue en soi pour transformer un nitrile en thioamide, puis isole le produit et le transforme éventuellement en un sel d'addition avec un acide.

7 - Procédé de préparation d'un produit selon la revendication 1 dans la formule duquel les symboles sont définis comme à la revendication 1 en A2), en B ou en C, caractérisé en ce que l'on hydrolyse un nitrile de formule générale :

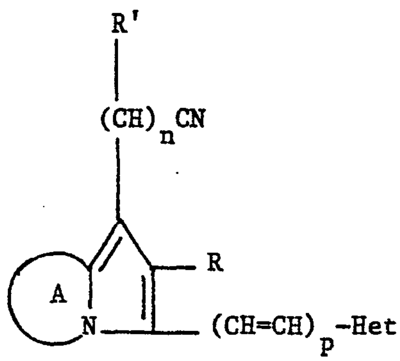

(IV)

dans laquelle les symboles ont les définitions correspondantes, par tout moyen connu de l'homme du métier pour transformer un nitrile en amide, puis isole le produit obtenu et le transforme éventuellement en un sel d'addition avec un acide.

8 - Procédé de préparation d'un produit selon la revendication 1, dans la formule duquel Z représente un atome d'oxygène, n est égal à zéro et les autres symboles sont définis comme à la revendication 1, caractérisé en ce que l'on fait agir un produit de formule générale :

$$R-C\equiv C-CO-Y \qquad (XVIII)$$

dans laquelle les symboles sont définis comme à la revendication 1, sur un produit de formule générale :

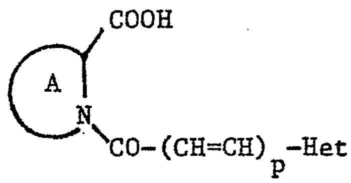

(VI)

dans laquelle les différents symboles sont définis comme à la revendication 1, puis isole le produit obtenu et le transforme éventuellement en un sel d'addition avec un acide.

9 - Procédé de préparation d'un produit selon la revendication 1 dans la formule duquel R représente un atome d'halogène, p est égal à 0 et les autres symboles sont définis comme à la revendication 1, caractérisé en ce que l'on procède à l'halogénation d'un produit selon la revendication 1 dans la formule duquel R représente un atome d'hydrogène, p est égal à 0 et les autres symboles sont définis comme à la revendication 1, puis isole le produit obtenu et le transforme éventuellement en un sel d'addition avec un acide.

10 - Médicament caractérisé en ce qu'il contient au moins un produit selon la revendication 1, éventuellement en association avec un ou plusieurs diluants ou adjuvants compatibles et pharmaceutiquement acceptables.

# REVENDICATIONS

1 - Procédé de préparation d'un nouveau dérivé orthocondensé du pyrrole, de formule générale :

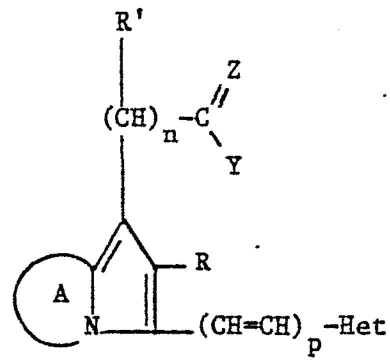

(I)

dans laquelle R' représente un atome d'hydrogène ou un radical alcoyle ou phényle éventuellement substitué par un atome d'halogène ou un radical alcoyle, alcoyloxy ou alcoylthio, Z représente un atome d'oxygène ou de soufre, p représente le nombre entier zéro ou 1 et

A - soit le symbole A représente un hétérocycle tel qu'avec le noyau pyrrole auquel il est condensé, il forme un cycle 1H,3H-pyrrolo [1,2-c] thiazole, dihydro-2,3 1H-pyrrolizine éventuellement substitué par un radical hydroxy, tétrahydro-5,6,7,8 indolizine, dihydro-1,2 4H-pyrrolo [1,2-c] thiazine-1,3 dihydro-2,3 pyrrolo [2,1-b] thiazole, 1H,3H-pyrrolo [1,2-c] oxazole, dihydro-1,2 4H-pyrrolo [1,2-c] oxazine-1,3 ou dihydro-2,3 pyrrolo [2,1-b] oxazole, n représente le nombre entier 0 ou 1, Het représente un radical pyridyle-3 ou thiazolyle-5 et

1) ou bien R représente un atome d'hydrogène ou d'halogène ou un radical alcoyle ou phényle éventuellement substitué par un atome d'halogène ou un radical alcoyle, alcoyloxy ou alcoylthio et Y représente un radical alcoyle ou phényle éventuellement substitué par un atome d'halogène ou un radical alcoyle, alcoyloxy ou alcoylthio, ou encore Y représente un radical de formule générale :

0147317

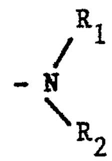

(II)

dans laquelle

- soit $R_1$ représente un atome d'hydrogène et $R_2$ représente un radical alcoyle non substitué, cycloalcoyle contenant 3 à 6 atomes de carbone, alcényle contenant 2 à 4 atomes de carbone, alcynyle contenant 3 ou 4 atomes de carbone, benzyle ou phényle éventuellement substitués par un atome d'halogène ou un radical alcoyle, alcoyloxy, alcoylthio, trifluorométhyle ou nitro, ou bien $R_2$ représente un radical adamantyle, pyridyle ou pyridylméthyle,

- soit $R_1$ et $R_2$ représentent tous deux un radical alcoyle non substitué,

- soit $R_1$ et $R_2$ forment avec l'atome d'azote auquel ils sont liés un radical phényl-4 pipérazinyle-1 dont la partie phényle peut être éventuellement substituée par un atome d'halogène ou un radical alcoyle, alcoyloxy, alcoylthio, trifluorométhyle ou nitro,

2) ou bien R représente un atome d'halogène ou un radical alcoyle ou phényle éventuellement substitué par un atome d'halogène ou un radical alcoyle , alcoyloxy ou alcoylthio, et Y représente un radical amino,

B - soit le symbole A représente un hétérocycle tel qu'avec le noyau pyrrole auquel il est condensé, il forme un cycle dihydro-2,3 1H-pyrrolizine substitué par un radical hydroxy, dihydro-2,3 pyrrolo [2,1-b] thiazole ou dihydro-2,3 pyrrolo [2,1-b] oxazole, R représente un atome d'hydrogène, Y représente un radical amino, n représente le nombre zéro ou 1 et Het représente un radical thiazolyle-5 ou pyridyle-3,

C - soit le symbole A représente un hétérocycle tel qu'avec le noyau pyrrole auquel il est condensé, il forme un cycle 1H,3H-pyrrolo [1,2-c] thiazole, dihydro-2,3 1H-pyrrolizine, tétrahydro-5,6,7,8 indolizine, dihydro-1,2 4H-pyrrolo [1,2-c] thiazine-1,3, 1H-3H-pyrrolo [1,2-c] oxazole ou dihydro-1,2 4H-pyrrolo [1,2-c] oxazine-1,3, R représente un atome d'hydrogène, Y représente un radical amino et

- soit Het représente le radical thiazolyle-5 et n est égal à zéro ou 1,

- soit Het représente le radical pyridyle-3 et n est égal à 1, étant entendu que, sauf mention spéciale, les radicaux alcoyle et portions alcoyle contiennent 1 à 4 atomes de carbone et sont en chaîne droite ou ramifiée,

ainsi que ses sels d'addition avec les acides, caractérisé en ce que,

i) pour la préparation d'un produit de formule générale (I) dans laquelle Z représente un atome d'oxygène, Y représente un radical alcoyle ou phényle éventuellement substitué par un atome d'halogène ou un radical alcoyle, alcoyloxy ou alcoylthio et les autres symboles sont définis comme précédemment, on fait agir un dérivé organomagnésien de formule générale :

$$Y'\text{-}MgX_1 \qquad (III)$$

dans laquelle Y' représente un radical alcoyle ou phényle éventuellement substitué par un atome d'halogène ou un radical alcoyle, alcoyloxy ou alcoylthio et $X_1$ représente un atome d'halogène, sur un nitrile de formule générale :

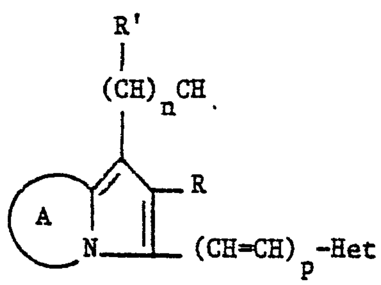

(IV)

dans laquelle les symboles sont définis comme précédemment, puis isole le produit obtenu et le transforme éventuellement en un sel d'addition avec un acide, ou en ce que,

ii) pour la préparation d'un produit de formule générale (I) dans laquelle les symboles sont définis comme précédemment, à l'exception pour Z de représenter un atome de soufre et pour Y de représenter un radical alcoyle ou phényle éventuellement substitué par un atome d'halogène ou un radical alcoyle, alcoyloxy ou alcoylthio, on fait réagir l'ammoniac ou une amine de formule générale :

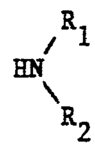 (XIV)

dans laquelle $R_1$ et $R_2$ sont définis comme précédemment sur un acide de formule générale :

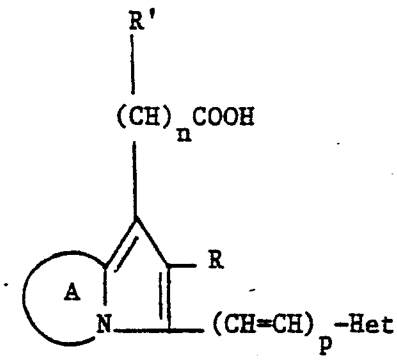 (XV)

dans laquelle les différents symboles sont définis comme précédemment, puis isole le produit obtenu et le transforme éventuellement en un sel d'addition avec un acide, ou en ce que,

iii) pour la préparation d'un produit de formule générale (I) dans laquelle Z représente un atome d'oxygène, Y représente un radical méthyle et les autres symboles sont définis comme précédemment , on fait agir l'éthoxymagnésien du malonate d'éthyle sur un halogénure de formule générale :

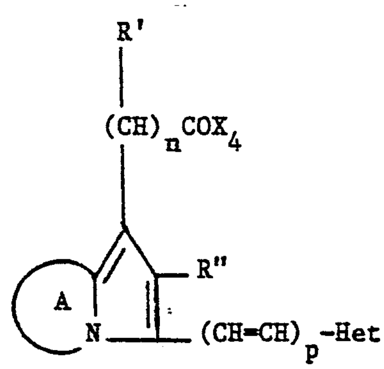 (XVI)

dans laquelle $X_4$ représente un atome d'halogène, R" a la définition donnée précédemment pour R, et les autres symboles sont définis comme précédemment, puis isole le produit et le transforme éventuellement en un sel d'addition avec un acide, ou en ce que,

iv) pour la préparation d'un produit de formule générale (I) dans laquelle Z représente un atome de soufre et les autres symboles sont définis comme précédemment, on procède à la thionation d'un produit de formule générale (I) dans laquelle Z représente un atome d'oxygène et les autres symboles sont définis comme précédemment, c'est-à-dire un produit de formule générale :

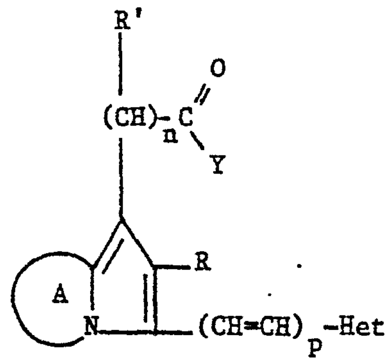

(XVII)

par toute méthode connue pour transformer un amide ou une cétone respectivement en thioamide ou thiocétone, puis isole le produit et le transforme éventuellement en un sel d'addition avec un acide, ou en ce que,

v) pour la préparation d'un produit de formule générale (I) dans laquelle Z représente un atome de soufre, Y représente un radical amino et R représente un radical alcoyle ou phényle éventuellement substitué par un atome d'halogène ou un radical alcoyle, alcoyloxy ou alcoylthio, on transforme en thioamide un nitrile défini comme précédemment en i) par toute méthode connue en soi pour transformer un nitrile en thioamide, puis isole le produit et le transforme éventuellement en un sel d'addition avec un acide, ou en ce que,

vi) pour la préparation d'un produit de formule générale (I) dans laquelle les symboles sont définis comme précédemment en A2), en B ou en C, on hydrolyse un nitrile de formule générale :

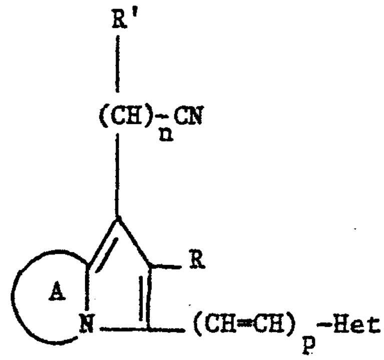

(IV)

dans laquelle les symboles ont les définitions correspondantes, par tout moyen connu de l'homme du métier pour transformer un nitrile en amide, puis isole le produit obtenu et le transforme éventuellement en un sel d'addition avec un acide, ou en ce que,

vii) pour la préparation d'un produit de formule générale (I), dans laquelle Z représente un atome d'oxygène, n est égal à zéro et les autres symboles sont définis comme précédemment, on fait agir un produit de formule générale :

$$R-C\equiv C-CO-Y \qquad (XVIII)$$

dans laquelle les symboles sont définis comme précédemment, sur un produit de formule générale :

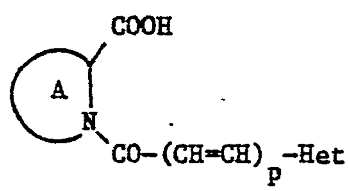

(VI)

dans laquelle les différents symboles sont définis comme précédemment, puis isole le produit obtenu et le transforme éventuellement en un sel d'addition avec un acide, ou en ce que,

viii) pour la préparation d'un produit de formule générale (I) dans laquelle R représente un atome d'halogène, p est égal à 0 et les autres symboles sont définis comme précédemment, on procède à l'halogénation d'un produit de formule générale (I) dans laquelle R représente un atome d'hydrogène, p est égal à 0 et les autres symboles sont définis comme précédemment, puis isole le produit obtenu et le transforme éventuellement en un sel d'addition avec un acide.